# EUROPEAN PATENT SPECIFICATION

(11) **EP 4 132 508 B1**
(45) Date of publication and mention of the grant of the patent: **12.11.2025**
(21) Application number: 21784228.5
(22) Date of filing: 08.04.2021
(51) Int. Cl.: C07D 333/38, C07D 409/12, A61K 31/38, A61P 35/00

(54) **NOVEL INHIBITORS OF HISTONE ACETYLTRANSFERASE P300/CBP FOR CANCER THERAPY**
NEUE INHIBITOREN DER HISTONACETYLTRANSFERASE P300/CBP ZUR KREBSTHERAPIE
NOUVEAUX INHIBITEURS DE L'HISTONE ACÉTYLTRANSFÉRASE P300/CBP POUR LA THÉRAPIE DU CANCER

(30) Priority: 09.04.2020 US 202063007711 P
(43) Date of publication of application: 15.02.2023
(73) Proprietor: Baylor College of Medicine, Houston, TX 77030-3498 (US)
(72) Inventor: SONG, Yongcheng, Houston, TX 77030 (US); WU, Fangrui, Houston, TX 77030 (US); NIE, Shen-You, Houston, TX 77030 (US); HUA, Yuanda, Houston, TX 77030 (US); LIN, Yi-Lun, Houston, TX 77030 (US); KAOCHAR, Salma, Houston, TX 77030 (US); MITSIADES, Nicholas, Houston, TX 77030 (US)
(74) Representative: Murgitroyd & Company
(86) International application number: PCT/US2021/026348
(87) International publication number: WO 2021/207469

(56) References cited:
- WO-A1-2020/210428
- WO-A1-2021/119447
- US-A1- 2006 122 229
- US-A1- 2011 306 539
- US-A1- 2018 000 821
- FANGRUI WU ET AL: "3-(Piperidin-4-ylmethoxy)pyridine Containing Compounds Are Potent Inhibitors of Lysine Specific Demethylase 1", JOURNAL OF MEDICINAL CHEMISTRY, vol. 59, no. 1, 14 January 2016 (2016-01-14), US, pages 253 - 263, XP055440785, ISSN: 0022-2623, DOI: 10.1021/acs.jmedchem.5b01361
- WU FANGRUI ET AL: "Discovery, Structure-Activity Relationship, and Biological Activity of Histone-Competitive Inhibitors of Histone Acetyltransferases P300/CBP", JOURNAL OF MEDICINAL CHEMISTRY, vol. 63, no. 9, 21 April 2020 (2020-04-21), US, pages 4716 - 4731, XP093141688, ISSN: 0022-2623, DOI: 10.1021/acs.jmedchem.9b02164
- MANZO F ET AL: "Histone acetyltransferase inhibitors and preclinical studies", EXPERT OPINION ON THERAPEUTIC PATENTS, TAYLOR & FRANCIS, GB, vol. 19, no. 6, 1 June 2009 (2009-06-01), pages 761 - 774, XP002616149, ISSN: 1354-3776, DOI: 10.1517/13543770902895727
- STEFANO VERNARECCI ET AL: "Tuning acetylated chromatin with HAT inhibitors: A novel tool for therapy", EPIGENETICS, vol. 5, no. 2, 16 February 2010 (2010-02-16), US, pages 105 - 111, XP055333750, ISSN: 1559-2294, DOI: 10.4161/epi.5.2.10942
- DATABASE PubChem Compound 26 April 2019 (2019-04-26), "2-Methylthiazole", XP055865180, retrieved from ncbi Database accession no. CID 383220112

## Description

### BACKGROUND

Histone acetylases, such as human p300 (E1A binding protein p300) and its paralog CBP (CREB (cAMP-response element binding protein) binding protein), are essential for many transcription factor-mediated gene transcription programs. Furthermore, p300 and CBP may contribute to the progression of numerous cancers. However, current inhibitors of p300 and CBP suffer from numerous limitations. Numerous embodiments of the present disclosure aim to address the aforementioned limitations. Fangrui Wu et al., Journal of Medicinal Chemistry, Volume 59, No.1, pages 253-263 disclose compounds as inhibitors of lysine specific demethylase 1. Stefano Vernarecci et al., Epigenetics, Volume 5, No. 2, pages 105-111 disclose examples of histone acetyltransferase inhibitors.

### SUMMARY

In a first aspect of the present invention, there is provided a composition comprising a compound, wherein the compound is selected from the group consisting of: 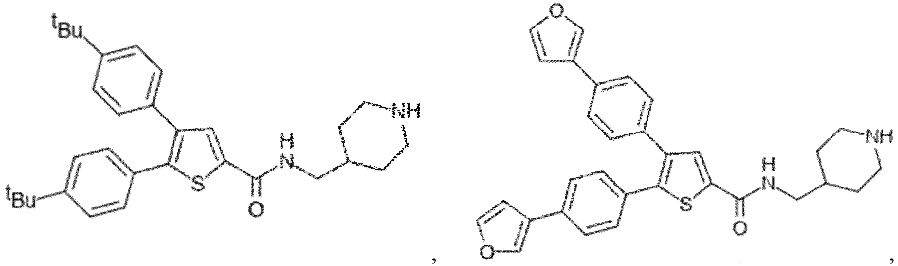 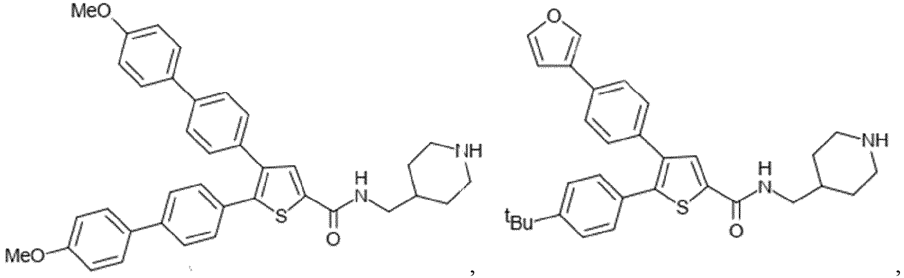 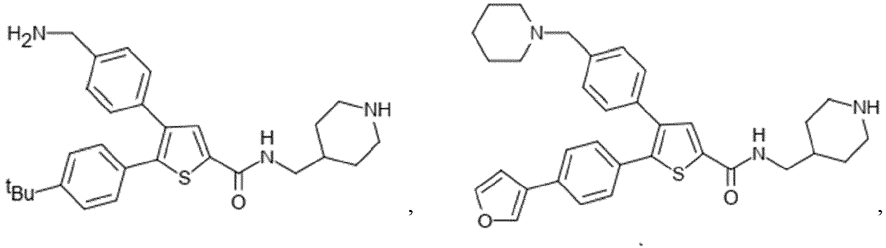 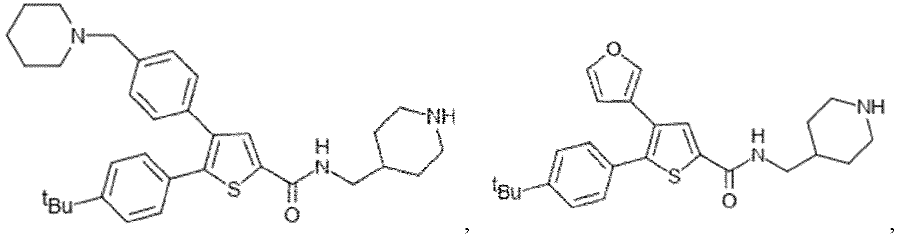 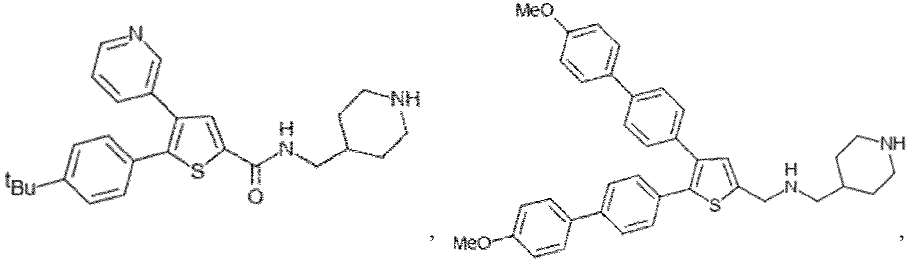 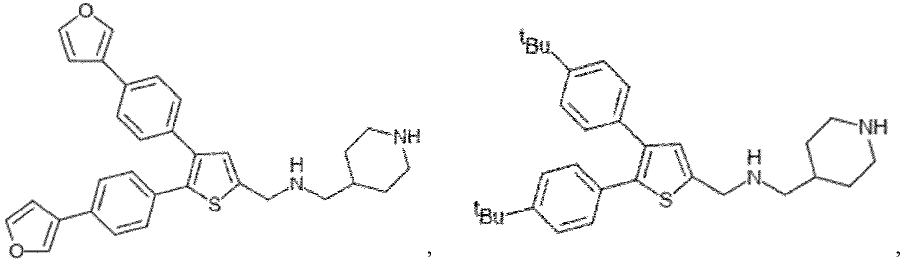 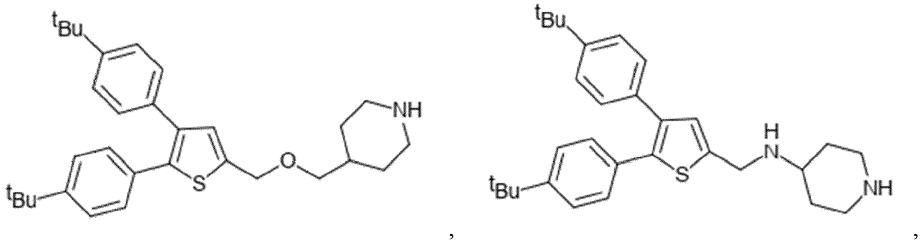 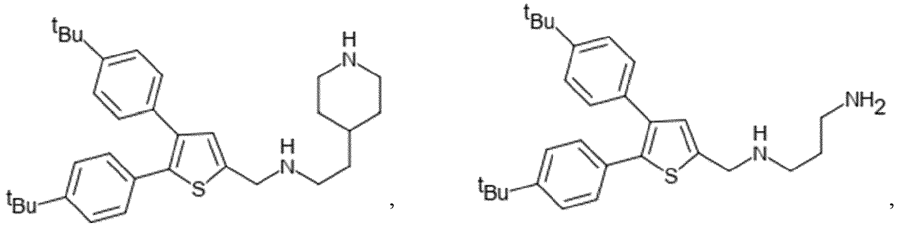 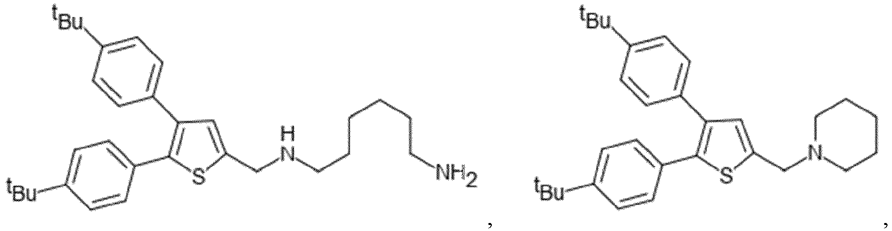 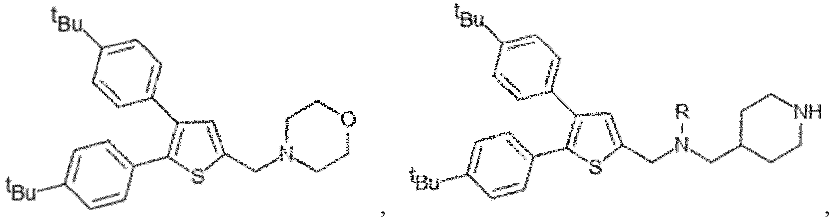 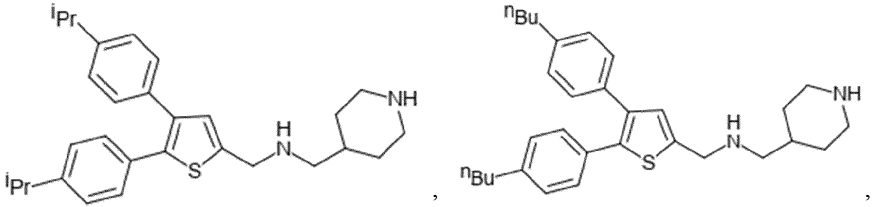 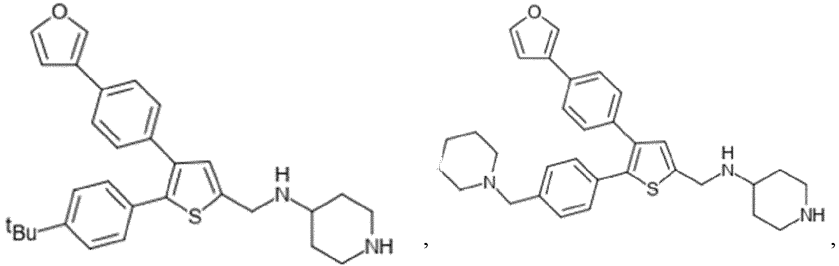 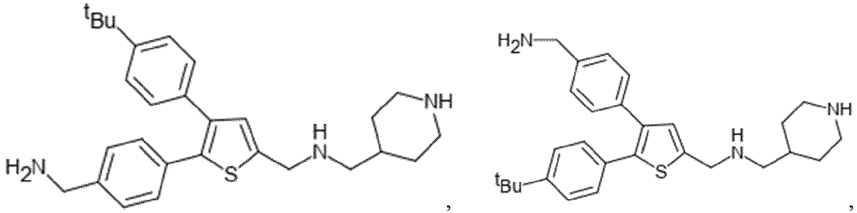 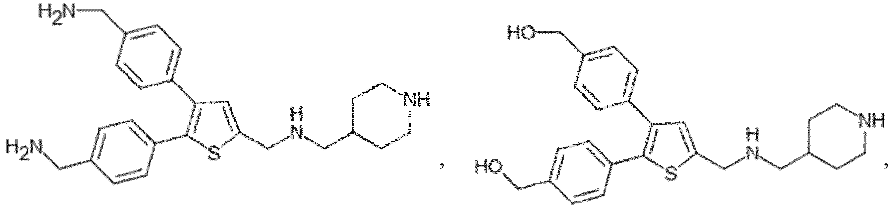 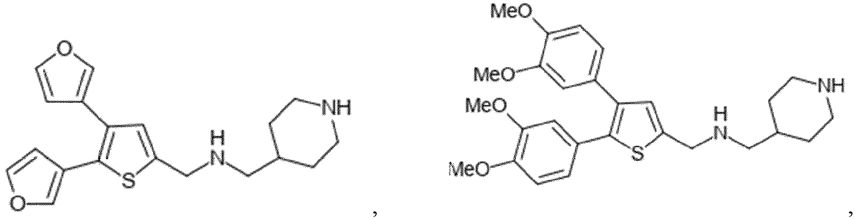 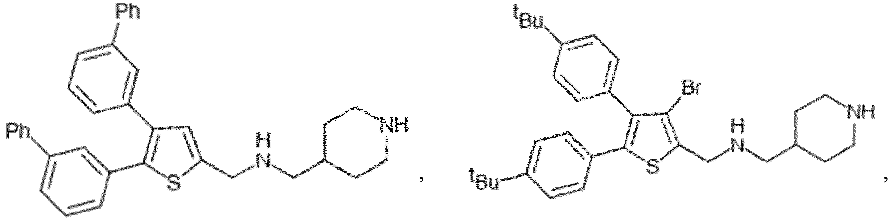 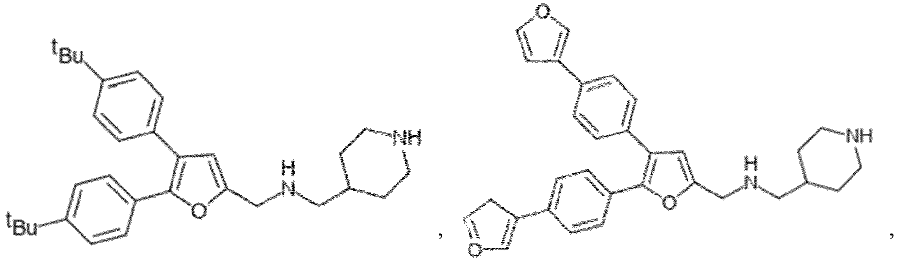 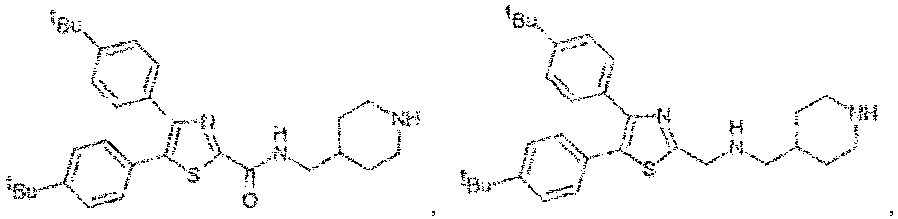 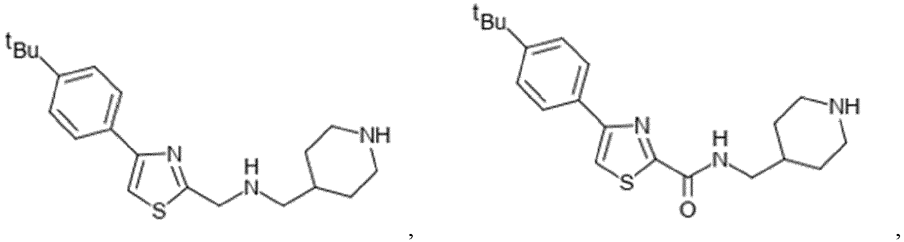 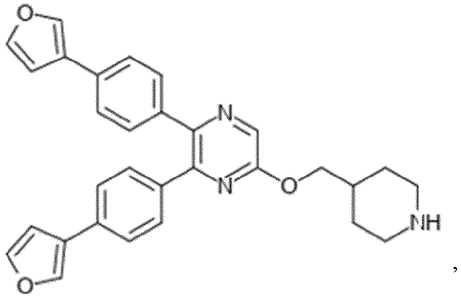 wherein R is Me or CHO.

In some embodiments, the composition of the present invention include the following

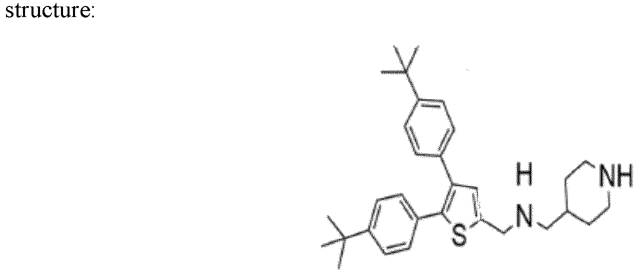

In some embodiments, the composition of the present invention include the following

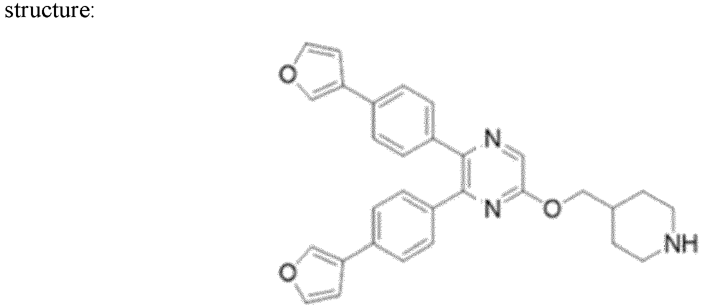

In some embodiments, the composition of the present invention comprises compounds that inhibit the histone acetyl transferase activity of a protein. In some embodiments, the protein is p300. In some embodiments, the protein is CBP.

In a further aspect of the present invention, there is provided a method of inhibiting the histone acetyl transferase activity of a protein (e.g., p300 and/or CBP) by exposing *in vitro* the protein to a composition that contains one or more of the compounds of the present invention.

In a further aspect of the present invention, there is provided a composition comprising a compound of claim 1 for use in treating a cancer in a subject. In some embodiments, the cancer to be treated includes, without limitation, cancers associated with p300 overexpression, cancers associated with CBP overexpression, and combinations thereof. In some embodiments, the cancer includes, without limitation, breast cancer, prostate cancer, pancreatic cancer, leukemia, acute myeloid leukemia, and combinations thereof. In some embodiments, the cancer is breast cancer.

### DESCRIPTION OF THE DRAWINGS

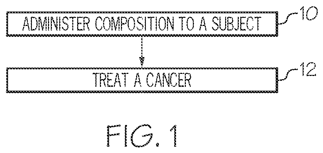

**FIG. 1** illustrates the compositions of the present disclosure for use in a method of treating a cancer in a subject through the administration of the compositions to the subject.

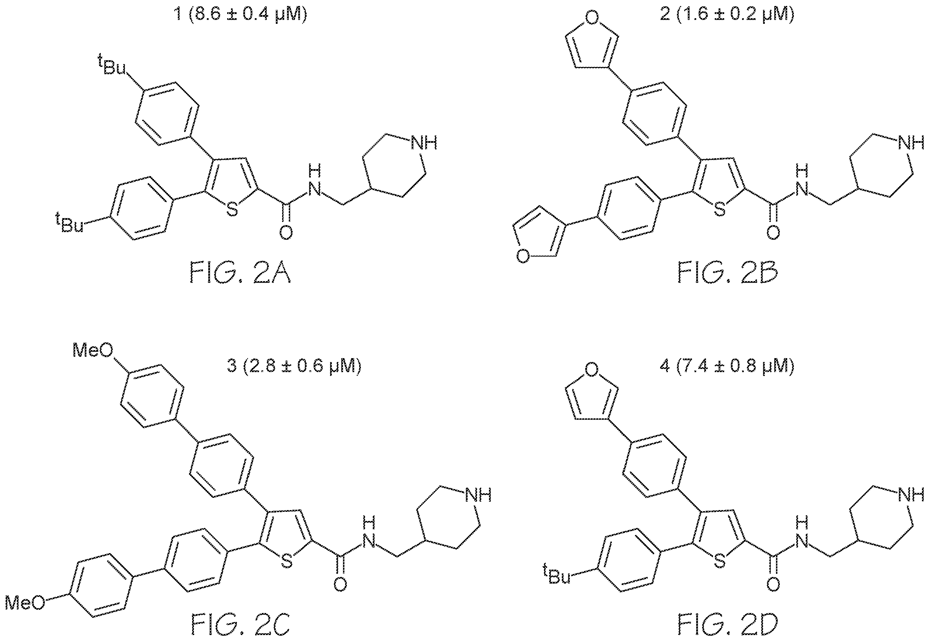

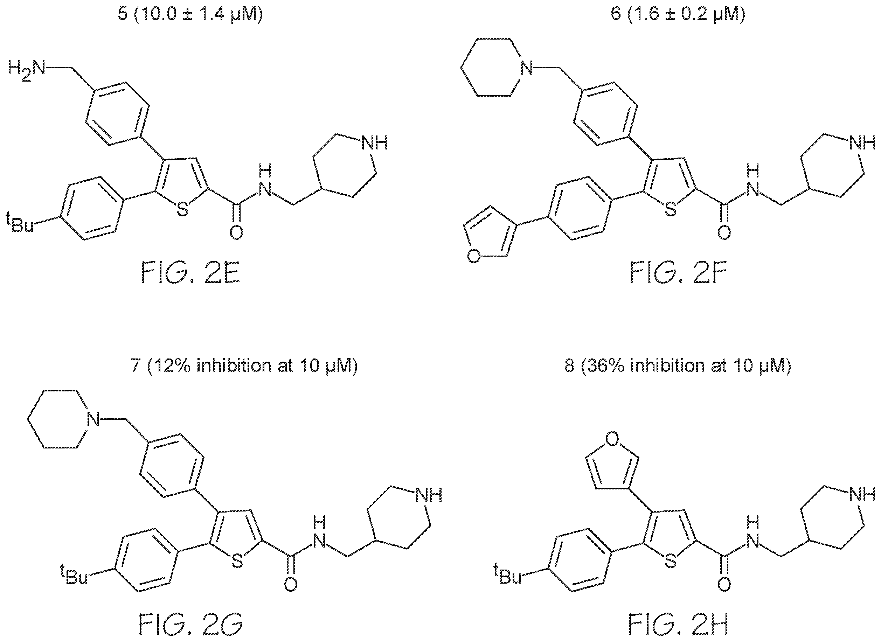

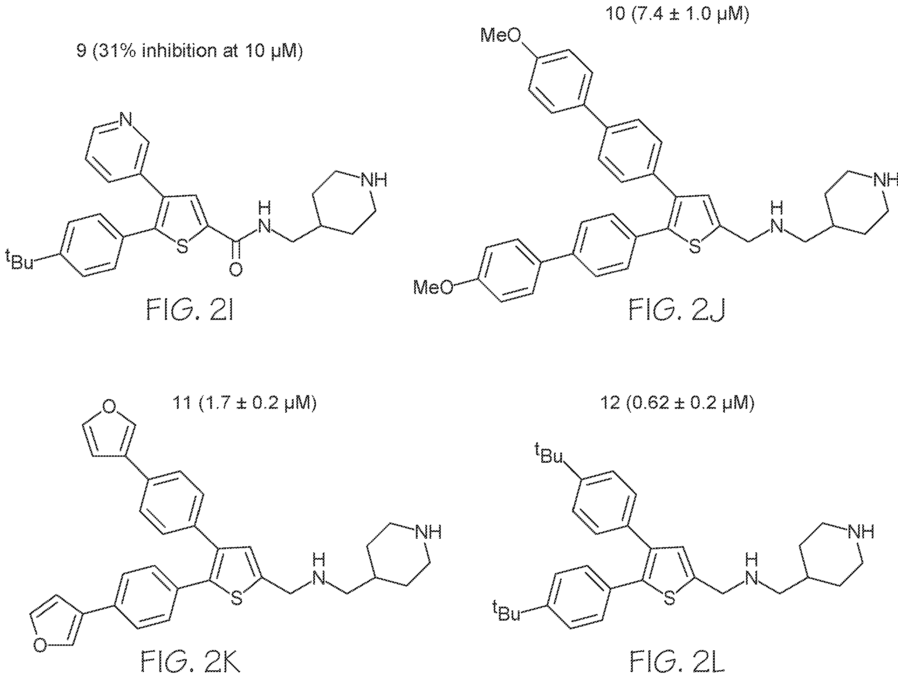

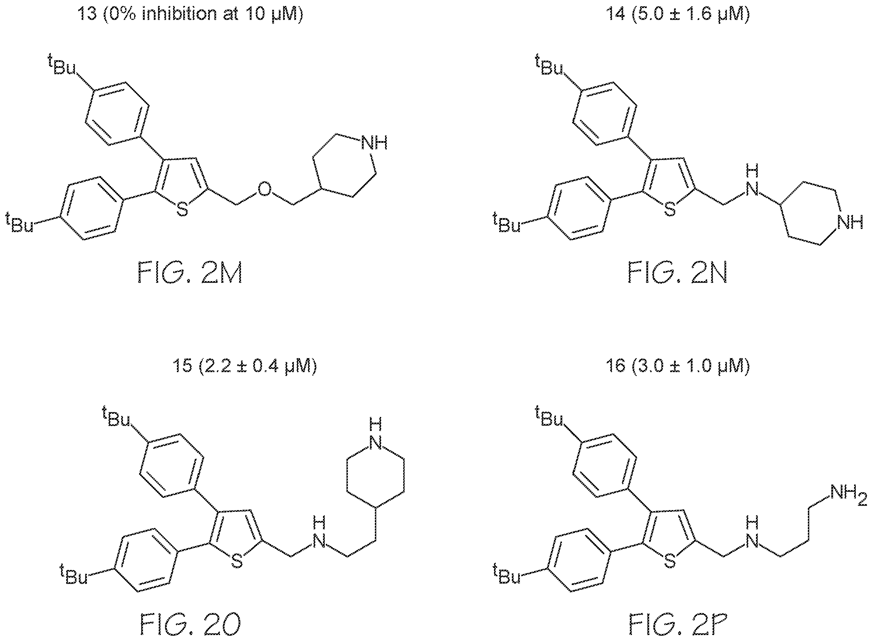

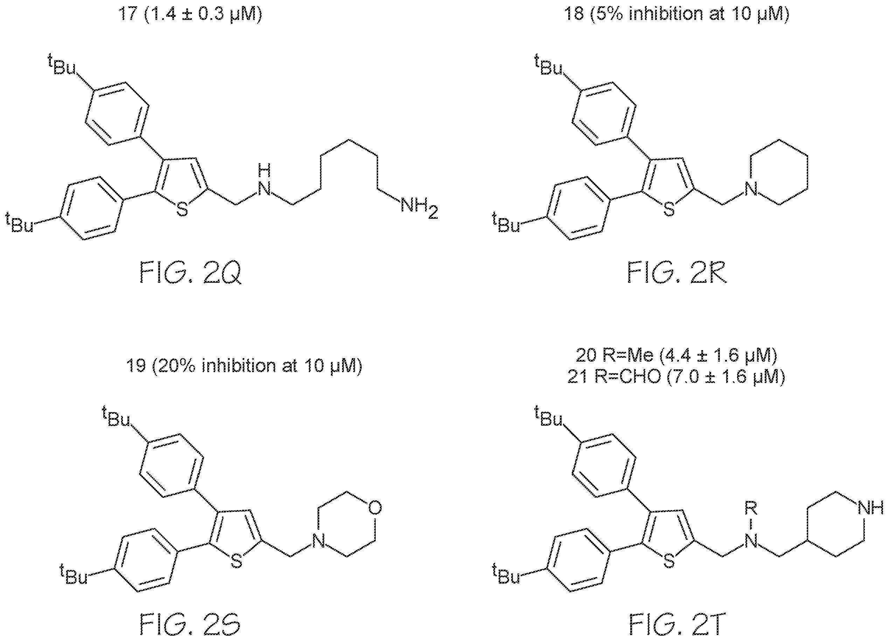

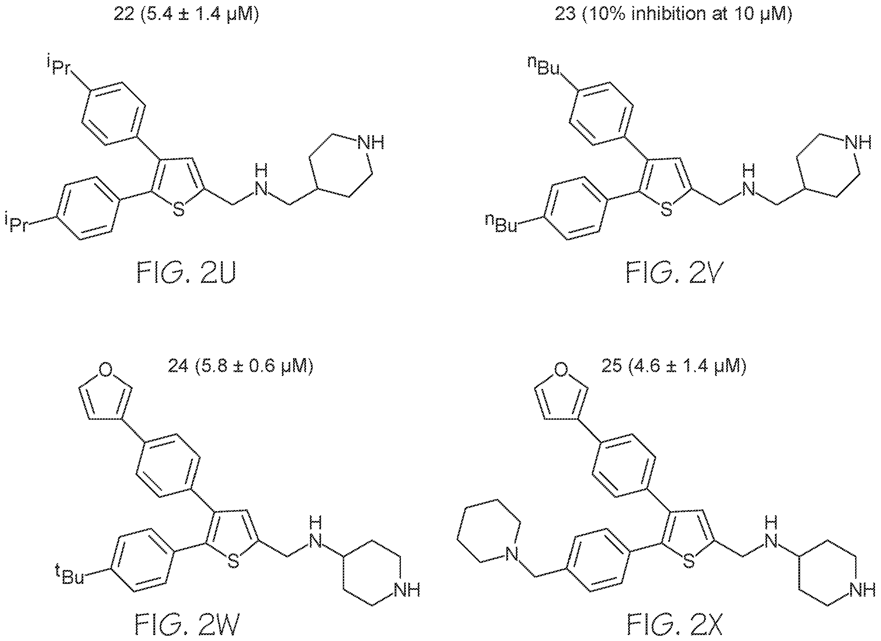

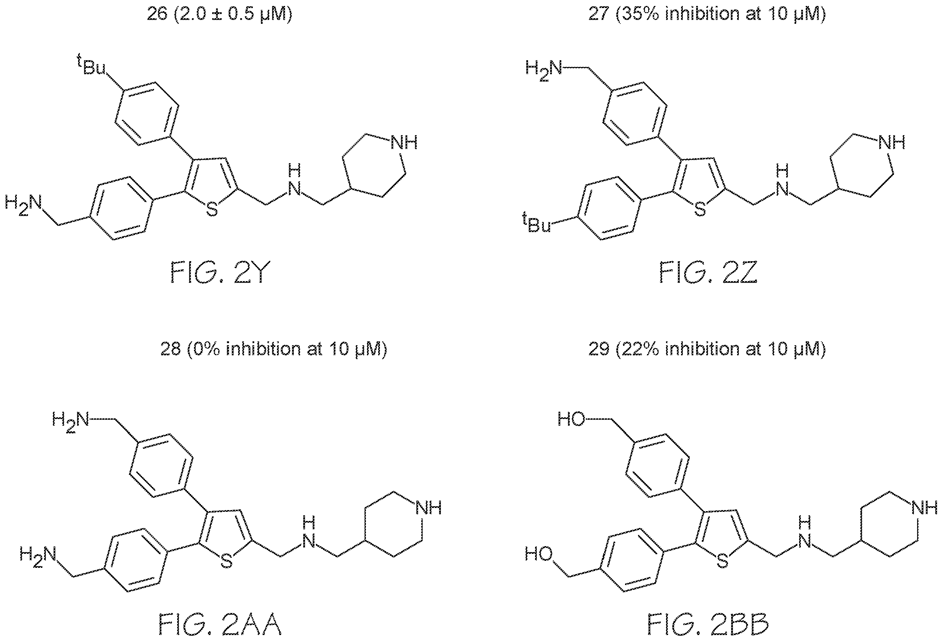

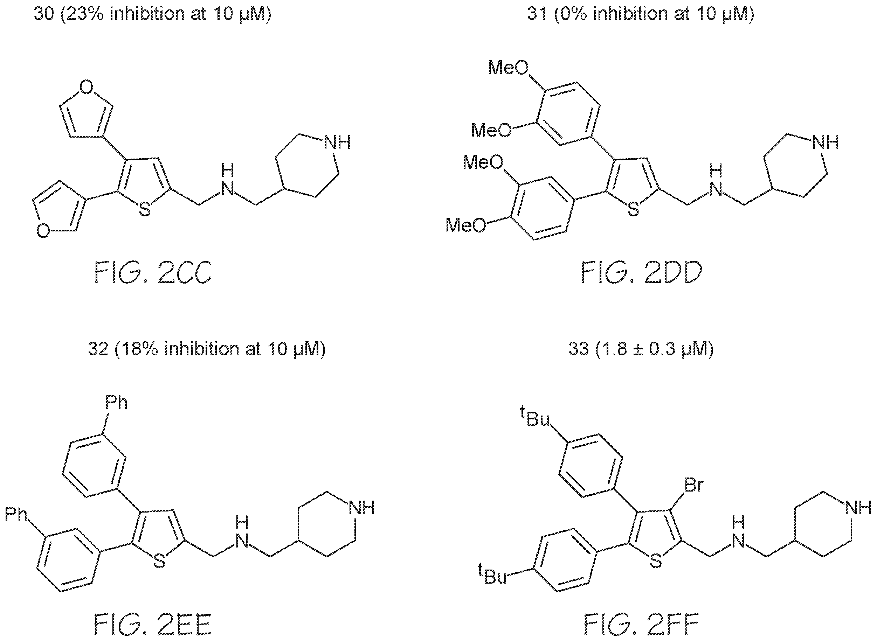

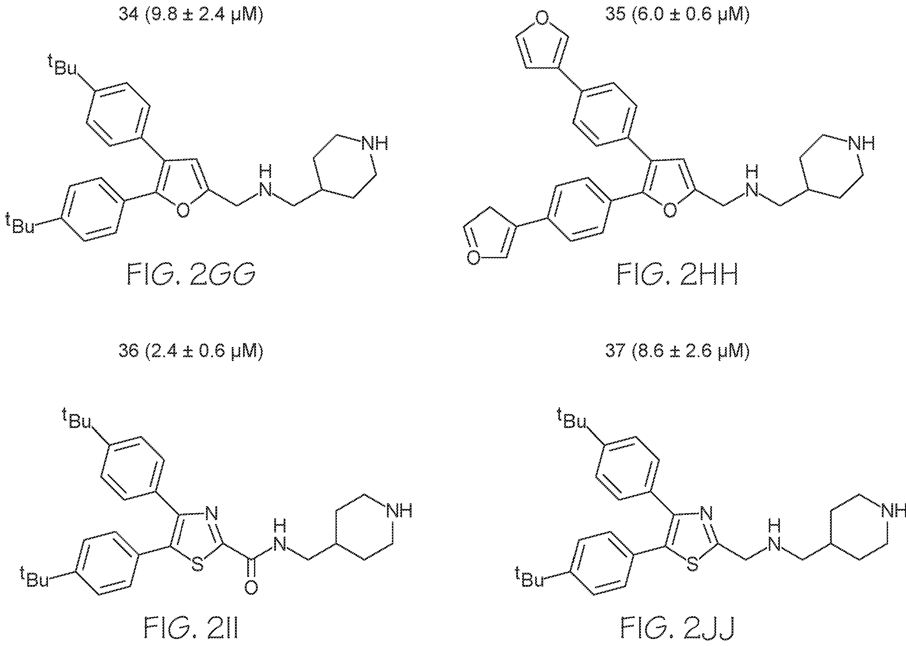

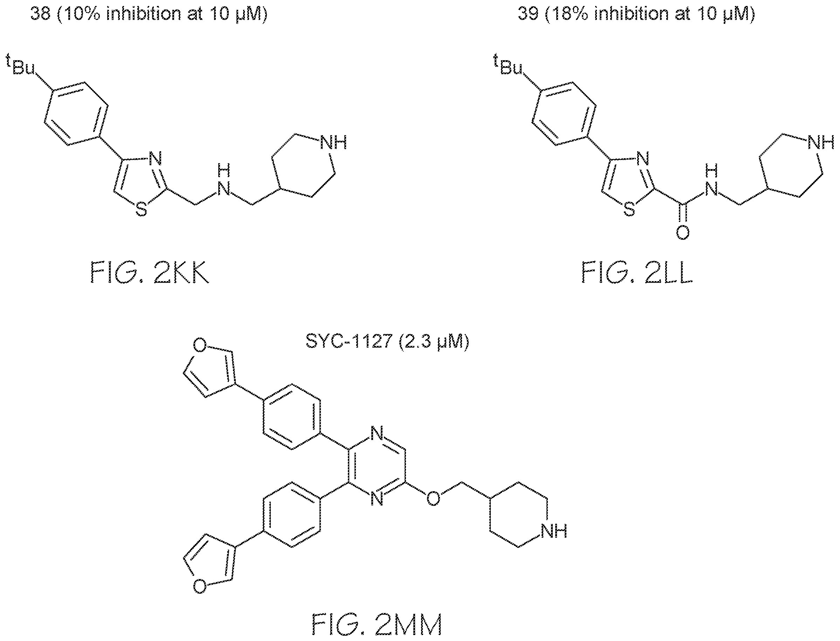

**FIGS. 2A-2MM** illustrate the structures of numerous compounds of the present invention and their inhibitory activities against p300 HAT.

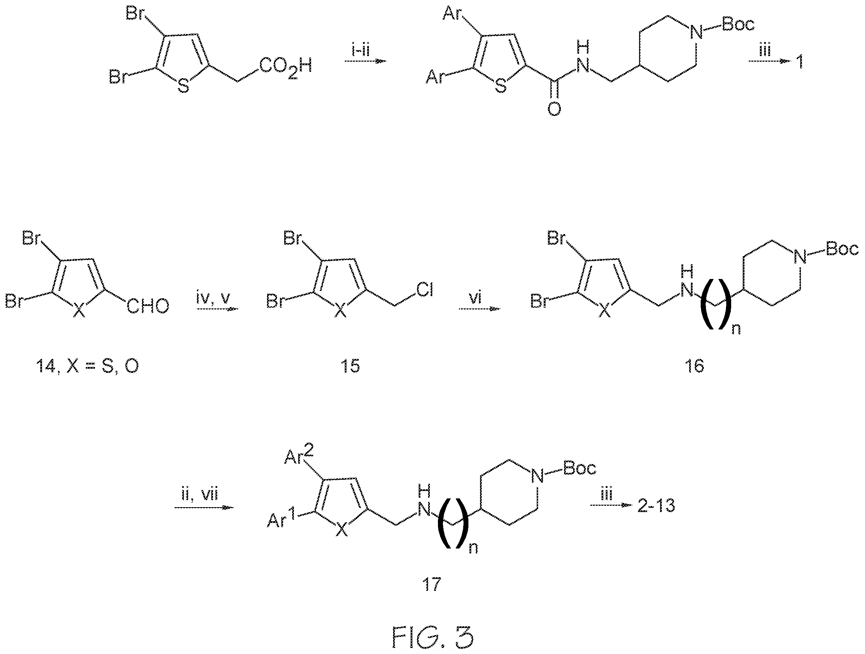

**FIG. 3** provide general synthetic methods for the compounds in **FIGS. 2A-2MM** of the present invention. *Reagents and conditions:* (i) 1-ethyl-3-(3-dimethylaminopropyl)carbodiimide, 1-hydroxybenzotriazole, 4-(aminomethyl)-1-BOC-piperidine, CH₂Cl₂, 96% yield; (ii) Ar-B(OH)₂, Pd(PPh₃)₄, Na₂CO₃, 80 °C, 80-90% yield; (iii) HCl in 1,4-dioxane, >90% yield; (iv) NaBH₄, MeOH; (v) cyanuric chloride, DMF, 81% yield for the 2 steps; (vi) 4-(aminomethyl)-1-BOC-piperidine or other amine analogs, K₂CO₃, DMF, 60-85% yield; (vii) Ar-B(OH)₂, Pd(PPh₃)₄, Na₂CO₃, 100 °C, 80-90% yield.

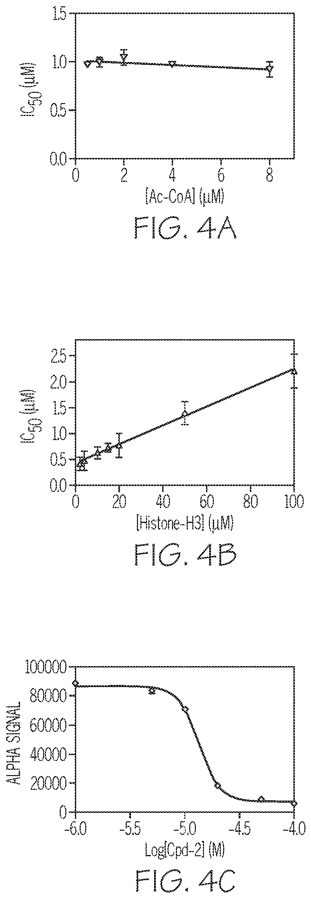

**FIGS. 4A-4C** illustrate that compound 12 is a competitive inhibitor of p300 HAT against the substrate histone. **FIGS. 4A-4B** show plots of IC₅₀ values of compound 12 versus increasing concentrations of Ac-CoA (**FIG. 4A**) and histone H3 (**FIG. 4B**). The results suggest that compound 12 is non-competitive against Ac-CoA and competitive against histone. **FIG. 4C** shows alpha assay results demonstrating that compound 12 can dose-dependently disrupt the binding between p300 HAT and histone H4.

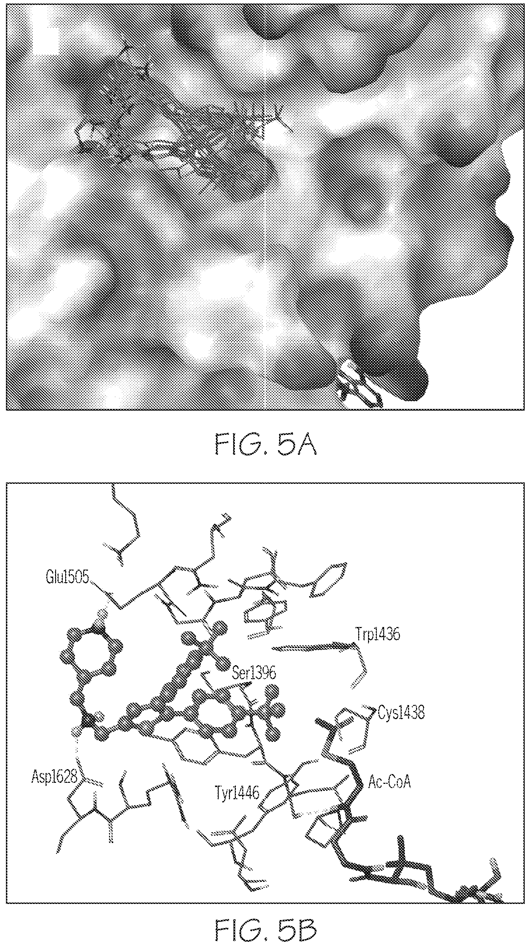

**FIGS. 5A-5B** show docking results for compound 12. **FIG. 5A** shows 10 docking conformations of compound 12 with lowest energies in the crystal structure of p300-HAT (shown as an electrostatic surface) in complex with Ac-CoA (as a tube model). **FIG. 5B** shows the lowest energy docking conformation of compound 12 (as a ball-and-stick model) with surrounding residues and Ac-CoA. Hydrogen bonds are shown as dotted lines.

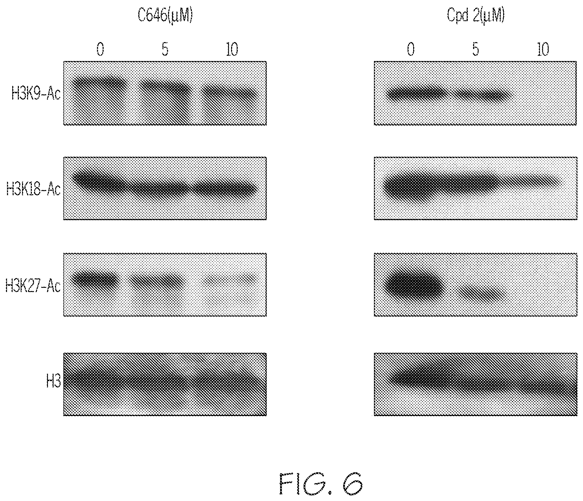

**FIG. 6** provides results illustrating that compound 12 and C646 inhibited cellular acetylation of H3K9, H3K18 and H3K27.

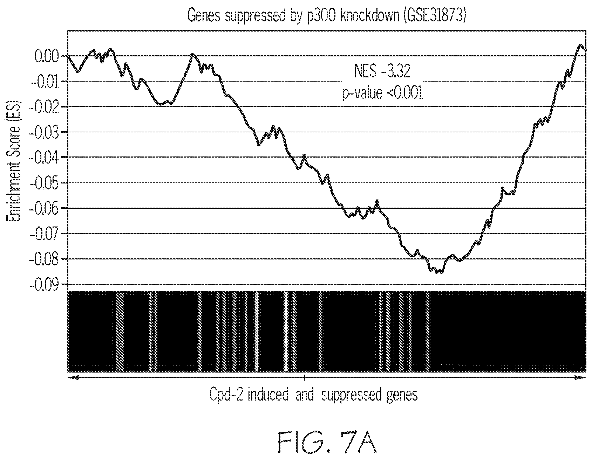

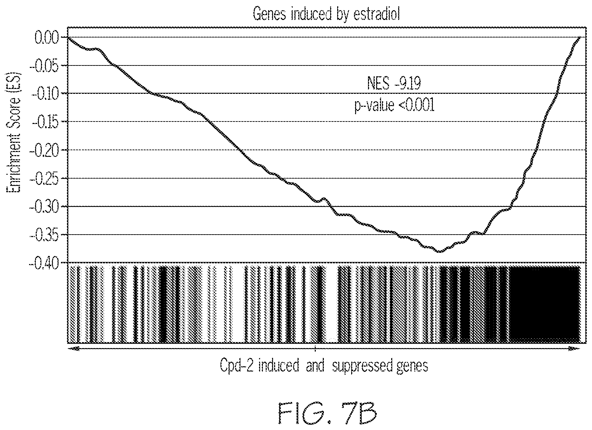

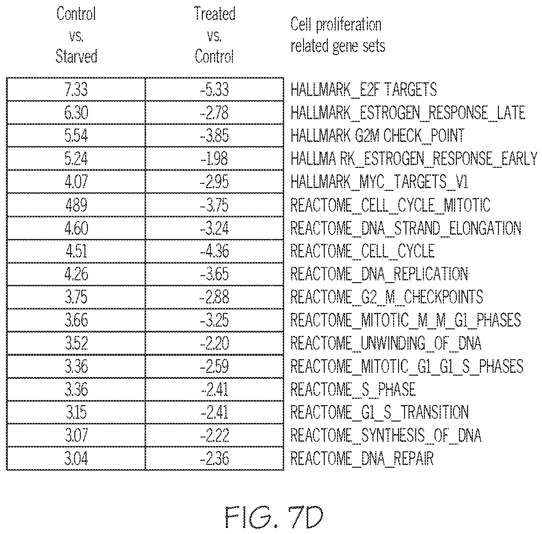

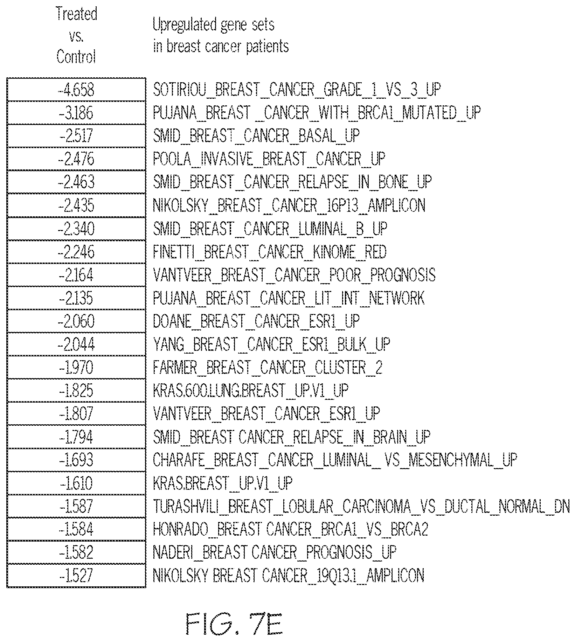

**FIGS. 7A-E** summarize gene set enrichment analysis (GSEA) results showing significant gene expression changes (*p* < 0.001) in ER+ MCF-7 cells by treatment with compound 12, as compared to the starved (without estradiol) or control (with estradiol) group. **FIG. 7A** summarizes results indicating that inhibitor treatment caused significant downregulation of genes that were suppressed by siRNA-mediated p300 knockdown (GSE31873). **FIGS. 7B-7E** summarize results showing that compound 12 counteracted estradiol in MCF-7 cells. Treatment with compound 12 significantly suppressed expression of genes that were upregulated by estradiol (**FIG. 7B**), and induced expression of genes that were downregulated by estradiol (**FIGS. 7D-E**). **FIGS. 7D-7E** show GSEA heatmaps of normalized enrichment scores (NES) for publicly available gene signatures (from the MSigDB database). **FIG. 7D** shows that inhibitor 12 caused significant downregulation of cancer-related gene sets that were induced by estradiol. **FIG. 7E** shows that inhibitor 12 suppressed expression of gene sets associated with breast cancer as well as the poor prognosis, progression, invasion and relapse of breast cancer.

### DETAILED DESCRIPTION

It is to be understood that both the foregoing general description and the following detailed description are illustrative and explanatory, and are not restrictive of the subject matter, as claimed. In this application, the use of the singular includes the plural, the word "a" or "an" means "at least one", and the use of "or" means "and/or", unless specifically stated otherwise. Furthermore, the use of the term "including", as well as other forms, such as "includes" and "included", is not limiting. Also, terms such as "element" or "component" encompass both elements or components comprising one unit and elements or components that include more than one unit unless specifically stated otherwise.

The section headings used herein are for organizational purposes and are not to be construed as limiting the subject matter described.

Histone acetylation is one of the most important post-translational modifications. Acetylation of the sidechain amino group of a histone lysine residue neutralizes its positive charge (under physiological pH) and renders a more open DNA conformation to facilitate binding of transcription factors as well as other proteins for gene transcription, DNA replication and repair. In addition, acetylated lysine can be recognized by a number of proteins (such as bromodomain-containing proteins) and serve as an anchor point in chromatin to form a transcription complex that regulates gene expression.

Human p300 (E1A binding protein p300) and its paralog CBP (CREB (cAMP-response element binding protein) binding protein) are large proteins with ~2,400 amino acids, containing multiple structured domains including cysteine-histidine rich 1 (CH1), CREB-binding KIX, Bromodomain, histone acetyltransferase (HAT), CH3, and steroid receptor coactivator interaction (SID) domains. These structured domains, which share a high homology between p300 and CBP, are connected with less conserved intrinsically disordered regions (IDR).

The CH1, KIX, CH3 and SID domains as well as IDR of p300/CBP are transactivation domains, which interact with a number of transcription factors (e.g., CREB, p53 and HIF-1) and transcription coactivators (e.g., steroid receptor coactivators). Biologically, p300/CBP not only acetylates a lysine residue of histone (e.g., histone H3 lysine 27, or H3K27) and certain transcription factors (e.g., p53 and c-Myc), it also serves as a hub protein to link and stabilize a transcription complex.

Previous studies have shown that HAT activity of p300/CBP is essential for many transcription factor-mediated gene transcription programs. For example, estrogen receptor (ER) or androgen receptor (AR) mediated gene transcription pathway is of importance in female or male development as well as in breast or prostate cancer, respectively. HAT activity of p300 has been found to be required for ER- or AR-mediated gene expression. In addition, HAT of p300/CBP is a potential drug target for cancer therapy. Although there have been debates on whether p300/CBP alone is a tumor suppressor or an oncogene, convincing evidence has shown that p300/CBP HAT is essential for breast and prostate cancer as well as other cancers with p300 overexpression or harboring a p300/CBP fusion oncogene.

To date, several chemotypes of p300/CBP HAT inhibitors have been reported. However, except for recently disclosed compound A-485, other compounds are not good probes for cellular or in vivo studies. For instance, many p300/CBP HAT inhibitors suffer from limited cell permeability or efficacy, which render them unfavorable for drug development or cell biology.

As such, a need exists for the development of more effective inhibitors of p300 and CBP for numerous applications, including applications as chemical probes for biological studies of p300/CBP and cancer therapeutics. Numerous embodiments of the present disclosure address the aforementioned needs.

### Compositions

The present disclosure pertains to compositions that include a compound disclosed herein for reference and compositions that include compounds of the present invention. The compound disclosed herein for reference includes a structure that includes, without limitation:

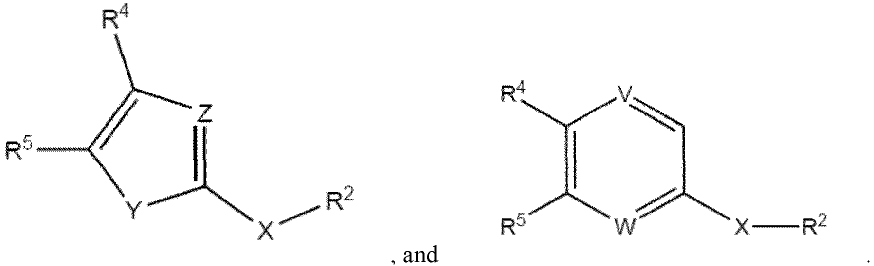

The structures of the compounds disclosed herein for reference can include numerous functional groups. For instance, in some embodiments, R₂, R₄, and R₅ can each independently include, without limitation, hydrogen, aromatic groups, phenyl groups, benzyl, furan groups, furanylphenyl groups, pyridine groups, pyridinylphenyl groups, biphenyl groups, piperidinylphenyl groups, pyrazole groups, piperidine groups, amine groups, alkyl amine groups, phenyl amine groups, phenyl alkyl amine groups, aniline groups, methyl piperidine groups, benzene groups, cyclohexane groups, methyl benzoate groups, benzyl piperidine groups, imidazole groups, piperidine amine groups, cyclic amine groups, cyclic aliphatic amine groups, cyclic piperidine-containing groups, 4-tertiary-butyl-phenyl (4-t-Bu-Ph), 4-isopropyl-phenyl (4-i-Pr-Ph), 4-Bu-Ph, 4-(furan-3-yl)-Ph, 4-methoxybiphenyl, 4-aminomethyl-Ph, 4-hydroxymethyl-Ph, piperidin-4-yl, piperidin-4-ylmethyl, 2-(piperidin-4-yl)ethyl, 4-(piperidin-1-ylmethyl)-Ph, piperazin-1-yl, 4-(piperazin-1-ylmethyl)-Ph, furan-3-yl, pyridin-3-yl, 4-methoxybiphenyl, 4-(furan-3-yl)-Ph, 3,4-dimethoxy-Ph, 3-biphenyl, 3-aminopropyl, 6-aminohexyl, morpholin-4-yl, derivatives thereof, and combinations thereof.

Y may include, without limitation, S, O, NH, N-A, CH₂, and CHA. A may represent a functional group. The functional group may include, without limitation, alkyl groups, alkene groups, alkyne groups, amide groups, amine groups, alkyl amine groups, alkyl amide groups, ether groups, ester groups, halogens (e.g., Br), derivatives thereof, and combinations thereof. Y may be S. Y may be O.

Z, V and W may each include, without limitation, N, CH, and CA. X may include, without limitation, alkyl groups, alkene groups, alkyne groups, amide groups, amine groups, alkyl amine groups, alkyl amide groups, ether groups, ester groups, O, CONH, CH₂NH, CH₂O, CH₂, CH₂N(Me), CH₂N(CHO), derivatives thereof, and combinations thereof.

R₄ may include, without limitation, hydrogen, aromatic groups, phenyl groups, benzyl, furan groups, furanylphenyl groups, pyridine groups, biphenyl groups, piperidinylphenyl groups, piperidine groups, phenyl amine groups, phenyl alkyl amine groups, benzene groups, benzyl piperidine groups, piperidine amine groups, 4-t-Bu-Ph, 4-i-Pr-Ph, 4-Bu-Ph, 4-(furan-3-yl)-Ph, 4-methoxybiphenyl, 4-aminomethyl-Ph, 4-hydroxymethyl-Ph, 4-(piperidin-1-ylmethyl)-Ph, piperazin-1-yl, 4-(piperazin-1-ylmethyl)-Ph, furan-3-yl, pyridin-3-yl, 4-methoxybiphenyl, 4-(furan-3-yl)-Ph, 3,4-dimethoxy-Ph, 3-biphenyl, derivatives thereof, and combinations thereof.

R₅ may include, without limitation, hydrogen, aromatic groups, phenyl groups, benzyl, furan groups, furanylphenyl groups, pyridine groups, pyridinylphenyl groups, biphenyl groups, piperidinylphenyl groups, piperidine groups, phenyl amine groups, phenyl alkyl amine groups, benzene groups, benzyl piperidine groups, piperidine amine groups, 4-*t*-Bu-Ph, 4-*i*-Pr-Ph, 4-Bu-Ph, 4-(furan-3-yl)-Ph, 4-methoxybiphenyl, 4-aminomethyl-Ph, 4-hydroxymethyl-Ph, 4-(piperidin-1-ylmethyl)-Ph, piperazin-1-yl, 4-(piperazin-1-ylmethyl)-Ph, furan-3-yl, pyridin-3-yl, 4-methoxybiphenyl, 4-(furan-3-yl)-Ph, 3,4-dimethoxy-Ph, 3-biphenyl, derivatives thereof, and combinations thereof.

X may include, without limitation, alkyl groups, amide groups, amine groups, alkyl amine groups, alkyl amide groups, ether groups, CONH, CH₂NH, CH₂O, CH₂, CH₂N(Me), CH₂N(CHO), derivatives thereof, and combinations thereof.

R₂ may include, without limitation, piperidine groups, amine groups, alkyl amine groups, methyl piperidine groups, cyclohexane groups, piperidine amine groups, cyclic amine groups, cyclic aliphatic amine groups, cyclic piperidine-containing groups, piperidin-4-yl, piperidin-4-ylmethyl, 2-(piperidin-4-yl)ethyl, piperazin-1-yl, 3-aminopropyl, 6-aminohexyl, morpholin-4-yl, derivatives thereof, and combinations thereof.

Y may be S or O. Z may be N or CH. V may be N. W may be N.

The compounds disclosed herein for reference of the present disclosure include the following structure:

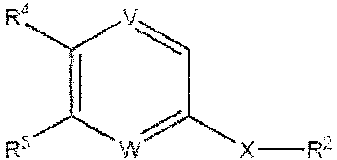

The compounds disclosed herein for reference include the following structure:

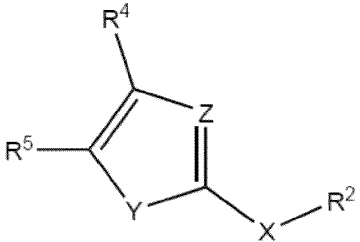

The compounds disclosed herein for reference include the following structure:

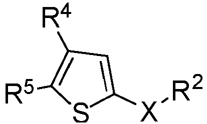

Each of R₄ and R₅ in the aforementioned structure may independently include, without limitation, 4-*t*-Bu-Ph, 4-*i*-Pr-Ph, and derivatives thereof. X in the aforementioned structure may include, without limitation, CONH, CH₂NH, and derivatives thereof. R₂ in the aforementioned structure may include piperidine groups, amine groups, alkyl amine groups, methyl piperidine groups, cyclohexane groups, piperidine amine groups, cyclic amine groups, cyclic aliphatic amine groups, cyclic piperidine-containing groups, piperidin-4-yl, piperidin-4-ylmethyl, 2-(piperidin-4-yl)ethyl, derivatives thereof, and combinations thereof.

The composition of the present invention include, without limitation, one or more of structures listed in **FIGS. 2A-2MM****.**

In some embodiments, the composition of the present invention include the following

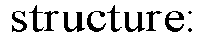

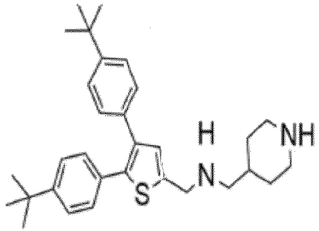

In some embodiments, the composition of the present invention include the following

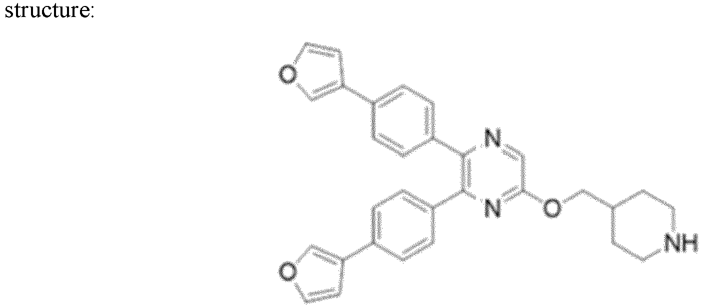

The compounds of the claimed composition have various advantageous properties and uses. For instance, in some embodiments, the composition comprises the compounds that inhibit the histone acetyl transferase activity of a protein. As such, in some embodiments, the composition of the present invention can be used to inhibit the histone acetyl transferase activity of a protein. In some embodiments, the protein is E1A binding protein p300 (p300). In some embodiments, the protein is CREB (cAMP-response element binding protein) binding protein (CBP).

In some embodiments, the compounds comprised in the claimed composition are competitive inhibitors of the histone acetyl transferase activity of a protein. In some embodiments, the compounds of the claimed composition are selective inhibitors of the histone acetyl transferase activity of a protein.

In some embodiments, the composition comprises compounds that inhibit the histone acetyl transferase activity of a protein with an IC₅₀ value ranging from 500 nm to 15 µM. In some embodiments, the compounds of the present disclosure inhibit the histone acetyl transferase activity of a protein with an IC₅₀ value ranging from 500 nm to 5 µM. In some embodiments, the compounds of the present disclosure inhibit the histone acetyl transferase activity of a protein with an IC₅₀ value ranging from 500 nm to 2 µM. In some embodiments, the compounds of the present disclosure inhibit the histone acetyl transferase activity of a protein with an IC₅₀ value ranging from 500 nm to 1 µM. In some embodiments, the compounds of the present disclosure inhibit the histone acetyl transferase activity of a protein with an IC₅₀ value of 620 nm.

Compositions that contain the compounds of the present disclosure may be in various forms. For instance, in some embodiments, the composition is associated with a delivery agent, such as a nanoparticle.

In some embodiments, the compositions of the present disclosure include at least one excipient agent. In some embodiments, the at least one excipient agent includes, without limitation, anti-adherents, binders, coatings, colors, disintegrants, flavors, glidants, lubricants, preservatives, sorbents, sweeteners, vehicles, or combinations thereof.

In some embodiments, the compositions of the present disclosure include at least one solubilizing agent. In some embodiments, the at least one solubilizing agent includes, without limitation, polyethylene glycol, glycerin, propylene glycol, ethanol, sorbitol, polyoxyethylated glycerides, polyoxyethylated oleic glycerides, polysorbates, sorbitan monooleate, hydroxypropyl-beta-cyclodextrin (HPCD), polyoxyl 40 hydrogenated castor oil, polyoxyl hydroxystearates, or combinations thereof.

### Methods of inhibiting histone acetyl transferase activities of a protein

Additional embodiments of the present invention pertain to *in vitro* methods of inhibiting the histone acetyl transferase activity of a protein. In some embodiments, the methods of the present invention include a step of exposing *in vitro* the protein to a composition that contains one or more of the compounds of the present invention.

In some embodiments, the *in vitro* methods of the present invention can be utilized to inhibit the histone acetyl transferase activity of p300. In some embodiments, the *in vitro* methods of the present invention can be utilized to inhibit the histone acetyl transferase activity of CBP.

As such, in some embodiments, the methods of the present disclosure can have various advantageous *in vitro* applications. For instance, in some embodiments, the methods of the present disclosure can be utilized to study biological activity *in vitro.* In more specific embodiments, the compounds of the present disclosure can be utilized as probes to study the enzymatic activity of proteins *in vitro.*

In some embodiments, the compositions of the present invention are provided for use in the treatment of cancer in a subject wherein the compositions may be exposed to a protein *in vivo* in a subject. In some embodiments, the composition is for administration by a method that includes, without limitation, oral administration, inhalation, subcutaneous administration, intravenous administration, intra-nasal administration, intra-dermal administration, trans-dermal administration, intraperitoneal administration, intramuscular administration, intrathecal injection, topical administration, central administration, peripheral administration, transdermal administration, intraarterial administration, intracranial administration, intraspinal administration, intranasal administration, intraocular administration, intratumor administration, intramuscular administration, intranasal administration, subcutaneous administration, intra- or trans-dermal administration, intravenous administration, topical administration, or combinations thereof.

The compositions of the present disclosure may be administered to various subjects. For instance, in some embodiments, the subject is a human being. In some embodiments, the subject is suffering from cancer. In some embodiments, the cancer includes, without limitation, cancers associated with p300 overexpression, cancers associated with CBP overexpression, and combinations thereof. In some embodiments, the cancer includes, without limitation, breast cancer, prostate cancer, pancreatic cancer, leukemia, acute myeloid leukemia, and combinations thereof. In some embodiments, the cancer includes breast cancer.

### Compositions For Use in treating cancer

Additional embodiments of the present invention pertain to compositions for use in treating a cancer in a subject. In some embodiments illustrated in **FIG. 1****,** a composition of the present invention is provided for use in the treatment of cancer in a subject (step 10) in order to treat the cancer (step 12).

In some embodiments, the compositions of the present invention are for administration through routes that include, without limitation, oral administration, inhalation, subcutaneous administration, intravenous administration, intra-nasal administration, intra-dermal administration, trans-dermal administration, intraperitoneal administration, intramuscular administration, intrathecal injection, topical administration, central administration, peripheral administration, transdermal administration, intraarterial administration, intracranial administration, intraspinal administration, intranasal administration, intraocular administration, intratumor administration, intramuscular administration, intranasal administration, subcutaneous administration, intra- or trans-dermal administration, intravenous administration, topical administration, or combinations thereof.

The compositions of the present invention can be utilized to treat cancer in numerous subjects. For instance, in some embodiments, the subject is a human being.

The compositions of the present invention can be utilized to treat numerous types of cancer. For instance, in some embodiments, the cancer includes, without limitation, cancers associated with p300 overexpression, cancers associated with CBP overexpression, and combinations thereof. In some embodiments, the cancer includes, without limitation, breast cancer, prostate cancer, pancreatic cancer, leukemia, acute myeloid leukemia, and combinations thereof. In some embodiments, the cancer is breast cancer.

### Additional Embodiments

Reference will now be made to more specific embodiments of the present disclosure and experimental results that provide support for such embodiments. However, Applicants note that the disclosure below is for illustrative purposes only.

### Example 1. Discovery and Biological Activity of Histone-Competitive Inhibitors of Histone Acetyltransferases P300/CBP

Histone acetyltransferase (HAT) p300 and its paralog CBP acetylate histone lysine sidechains play critical roles in regulating gene transcription. The HAT domain of p300/CBP is also a potential drug target for cancer. In this Example, Applicant disclose small-molecule inhibitors of p300/CBP HAT. Biochemical and cell-based biological assays were performed to characterize the activities of these compounds.

A novel series of small molecule inhibitors of p300/CBP HAT with IC₅₀ values as low as 620 nM were discovered and characterized. The most potent inhibitor was found to be competitive against the substrate histone, showing a different mode of action from previously reported inhibitors. The inhibitor exhibited a high selectivity for p300/CBP, without significant activity against two other classes of HATs in human. The molecule inhibited cellular acetylation of several histone lysine residues and had strong activity with EC₅₀ of 1-3 µM against proliferation of several tumor cell lines in which p300 HAT is of importance.

Gene expression profiling in estrogen receptor (ER)-positive breast cancer MCF-7 cells showed that treatment with the inhibitor recapitulated siRNA-mediated p300 knockdown, inhibited ER-mediated gene transcription, and suppressed expression of numerous cancer-related gene signatures. The results in this Example demonstrate that the small molecule inhibitor are not only a useful probe for biological studies of p300/CBP HAT, but also a pharmacological lead for further drug development targeting cancer.

### Example 1.1. Inhibitor discovery

Applicant reports the discovery, structure activity relationships (SAR), biochemical and biological activities of a novel series of small molecule inhibitors of p300/CBP HAT that are competitive against the histone substrate. A biochemical assay to determine the activity and inhibition of recombinant HAT domain of human p300 was developed, using the substrate histone H3 (1-21) peptide and the ³H-labeled cofactor Ac-CoA.

Applicant screened a proprietary compound library containing ~300 compounds, which were synthesized during the past few years for structure-activity relationship (SAR) studies of lysine specific demethylase 1 (LSD1), whose substrate is methylated histone H3 lysine 4 (H3K4). Thiophene-2-carbamide compound **1** (**FIG. 2A**) was found to be a novel inhibitor of p300 HAT with IC₅₀ (concentration at which the enzyme activity is inhibited by 50%) value of 8.6 µM.

### Example 1.2. Synthesis

More derivatives of compound **1** were synthesized and tested for their activities against p300 HAT. General methods for synthesis of the compounds in **FIGS. 2A-2MM** are shown in **FIG. 3****.** Synthesis of compound **1** was started with a standard amide formation reaction between 4,5-dibromo-thiophene-2-carboxylic acid and 1-*tert*-butyloxycarbonyl (BOC) protected 4-(aminomethyl)piperidine. The 5- and 4-Br groups of the amide thus obtained can undergo two Suzuki coupling reactions to give, upon deprotection of BOC, compound **1.** For compounds **11-16** and **20-23,** the 2-CHO group of 4,5-dibromothiophene-2-carbaldehyde (**14**, X = S) was reduced to an alcohol, followed by conversion to -CH₂Cl by treatment with cyanuric chloride. The -Cl of the product **15** was substituted with an amine and the resulting compound **16** was subjected to two Suzuki coupling reactions with different aryl boronic acids with a high selectivity (23): 5-Br was reacted first at a lower temperature of 80 °C, followed by 4-Br at 100 °C. Upon deprotection of BOC, the target thiophene compounds **11-16** and **20-23** were obtained. Compound **34** was synthesized similarly from the furan **14** (X = O).

### Example 1.3. Structure-activity relationship

Structures and inhibitory activities against p300 HAT of 13 representative compounds are shown in **FIG. 3****.** Compound **12** by replacing the amide (-CONH-) group in the 2-substituent of **1** with a secondary amine (-CH₂NH-) linkage was found to be a potent inhibitor with an IC₅₀ of 620 nM, while changing to a -CH₂O- linkage in compound **13** resulted in a complete loss of inhibitory activity. Compound **14** or **15,** whose 2-substituent is one -CH₂- shorter or longer than that of compound **12,** respectively, was found to possess ~8- or 3-fold activity reduction (IC₅₀ = 5.0 or 2.2 µM). Compound **16** with a linear 2-sidechain showed significantly reduced inhibitory activity (IC₅₀ = 3.0 µM), as compared to compound **12** with a more hydrophobic, cyclic piperidine-containing group. Loss of activity for a compound bearing a shorter piperazine ring suggests a longer and cyclic amine such as that in compound **12** is more favored. Moreover, methylation and formylation of the middle -NH- group in compound **12** produced compounds **20** and **21,** respectively, which were found to have less inhibitory activities with IC₅₀ values of 4.4 and 7.0 µM.

Replacing the two *tert*-butyl groups in compound **12** with less bulky isopropyl groups in compound **22** (IC₅₀ = 5.4 µM) led to ~8× activity reduction. Loss of activity for compound **23** with two *n*-butyl groups at these positions showed that longer alkyl substituents are disfavored. Changing to furan groups in compound **11** (IC₅₀ = 1.7 µM) also slightly reduced the inhibitory activity. In addition, compound **34** with a central furan core (IC₅₀ = 9.8 µM) was found to be considerably less active than the thiophene compound **12.**

### Example 1.4. Compound 2 is competitive against histone

Next, Applicant performed steady-state enzyme kinetics studies to investigate the mode of inhibition for the most potent inhibitor **12.** Inhibitory activities of compound **12** were determined with varying concentrations of the cofactor Ac-CoA and the substrate histone. As shown in **FIG. 4A****,** IC₅₀ values of **12** did not change with increasing concentrations of Ac-CoA (from 0.5 - 8 µM, or 0.07-1.2× *K*ₘ (24)), indicating compound **12** is likely a non-competitive inhibitor against the enzyme cofactor. Its inhibitory activities were found to be reduced in a linear manner (**FIG. 4A**) when increasing concentrations of the substrate histone peptide (2 - 100 µM) were used in the assay, suggesting compound **12** is a competitive inhibitor against the substrate histone.

Next, Applicant used a reported Alpha (amplified luminescent proximity homogeneous assay) assay to confirm this finding. Upon excitation by a laser beam (680 nm), histone H4 coated donor beads generate singlet oxygen radicals, which can travel for a very short distance (<200 nm) in the solution and cannot reach the free acceptor beads. The binding between the histone peptide and p300 HAT brings the donor and acceptor beads together, which allows the radicals to activate the p300 HAT coated acceptor beads and produce luminescence at 570 nm (**FIG. 4B**). Use of histone H4 is because the assay with histone H3 did not produce a luminescence signal, presumably due to a weaker binding between p300 HAT and histone H3.

As shown in **FIG. 4C****,** compound **12** can disrupt the binding between the protein and histone H4, and reduce the luminescence signal in a dose-dependent manner (IC₅₀ = 13.5 µM), showing the inhibitor is competitive against the substrate histone. This is in contrast to Ac-CoA competitive inhibitor A-485, which did not affect the binding between p300 HAT and histone H4(16). These results are of interest in that due to a different mode of inhibition, histone-competitive inhibitor **12** might have a different cellular activity or selectivity, as compared to Ac-CoA-competitive inhibitors A-485 and C646.

### Example 1.5. Docking studies

Next, Applicant performed molecular modeling to find possible binding structures of the most potent inhibitor **12** in p300 HAT. The program Glide in Schrödinger small-molecule drug discovery software suite was used for the docking studies, with the crystal structure of human p300 HAT in complex with Ac-CoA (PDB code 4PZS) (25) as a template.

Since compound **12** is a competitive inhibitor against the substrate histone, Ac-CoA was treated as an integrate part of the protein. Upon protein structure preparation as well as docking grid generation, compound **12** was docked into the protein-Ac-CoA complex and the results are shown in **FIGS. 5A** and **5B****.** The inhibitor can be favorably docked into the protein, as the 10 distinct docking conformations of compound **12** with the lowest energies exhibit similar binding features (**FIG. 5A**).

One of the *tert*-butyl groups of these conformations is predicted to insert into and have favorable hydrophobic interactions with the so-called "lysine channel", through which the histone lysine sidechain inserts into p300 HAT, attacks the carbonyl of Ac-CoA, and gets acetylated. Residues Trp1436, Cys1438, Tyr1446 and Ser1396 form the lysine channel of p300 HAT (**FIG. 5B**).

As shown in **FIG. 5B****,** the *tert*-butyl group is predicted to be ~3.5 Å from these residues with favorable van de Waals interactions. The central thiophene core and the two *para-tert-*butylphenyl groups of these structures have extensive hydrophobic interactions with either upper or lower surface of the protein (**FIGS. 5A** and **5B**). In addition, the positively charged, two amino groups of many of these docking structures form hydrogen bonds and have favorable electrostatic interactions with Asp1628 and Glu1505, as exemplified in **FIG. 5B****.** These favorable binding features are consistent with the strong inhibitory activity of compound **12.**

Moreover, the docking results might be used to rationalize some of the observed SARs. For example, hydrogen bond/electrostatic interactions between the middle -NH- and Asp1628 are predicted to contribute significantly to the binding of compound **12.** This could explain the loss of activity for compound **13** with an -O- linkage, which does not interact with the (negatively charged) Asp1628 sidechain. In addition, compound **1** is a good inhibitor, which might be due to favorable interactions between its -CONH- group and Asp1628.

### Example 1.6. Enzyme selectivity

Humans have three classes of histone/protein lysine acetyltransferases with distinct conserved motifs and structures, including p300/CBP, Gcn5-related N-acetyltransferase (GNAT) and MYST (MOZ, Ybf2, Sas2 and Tip60) family of HATs. Compound **12** was tested for its activities against selected HATs from these three classes of HATs. CBP is a homolog of p300. The HAT domains of CBP and p300 exhibit 87% identity in sequence. PCAF (p300/CBP associating factor) is a member of the GNAT family, while Myst3 belongs to the MYST family of HATs.

P300/CBP HATs have a distinct sequence and 3-dimensional structure from the GNAT and MYST HAT proteins. As shown in Table 1, compound **12** was found to be also a strong inhibitor of human CBP HAT with an IC₅₀ of 1.2 µM, comparable to its IC₅₀ against p300 HAT. However, compound **12** did not significantly inhibit the activity of PCAF and Myst3 even at 20 µM, showing a high selectivity for p300/CBP HAT.

**Table 1. Inhibitory activities of compound 12 against HATs.**

| | IC₅₀ (µM) |
|---|---|
| P300-HAT | 0.62 ± 0.12 |
| CBP-HAT | 1.2 ± 0.13 |
| PCAF | >20 (<10% inhibition at 20 µM) |
| Myst3 | >20 (<10% inhibition at 20 µM) |

### Example 1.7. Inhibition of cellular histone acetylation

Ability of compound **12** to inhibit cellular p300/CBP HAT was evaluated, together with a known inhibitor C646 as a comparison. Kasumi-1 leukemia cells were treated with increasing concentrations of these compounds for 12h. Histone was extracted and subjected to electrophoresis separation and Western blot staining to determine levels of acetylation at various lysine residues. As shown in **FIG. 6****,** compound **12** was found to significantly inhibit cellular acetylation of histone H3K9, H3K18 and H3K27 at 5 and 10 µM, in a dose dependent manner. H3K27 seems to be the most sensitive cellular substrate of p300 HAT. These results are consistent with previous studies of known inhibitors A-485 and C646. As compared to C646, compound **12** appeared to exhibit more potent cellular activities at these lysine residues. This might be due to different mode of inhibition or cell permeability for the two compounds.

### Example 1.8. Inhibition of tumor cell proliferation

Activity of compound **12** was tested against proliferation of several tumor cell lines in which p300/CBP HAT is of importance. First, ER-mediated transcription is critical to ER+ breast cancer. It is an ordered, stepwise assembly of ER and coactivator proteins for gene expression. Upon binding with an estrogen molecule in cytoplasm, ER undergoes conformational changes, forms a homodimer, and is translocated into the nucleus, where ER binds to estrogen response element, a short segment of DNA within a gene promoter. Next, a steroid receptor coactivator (SRC) protein binds to ER, followed by recruitment of p300 through its SRC-binding SID domain.

P300 HAT activity is required for the ER-mediated gene expression, because a mutant p300 without the enzyme function failed to activate it. Given the critical role of p300-mediated histone acetylation in the ER signaling pathway, compound **12** was assessed for its activity against the ER⁺ breast cancer cell line MCF-7. In addition, ER antagonist tamoxifen is widely used in the clinic to treat ER⁺ breast cancer by suppressing ER mediated gene transcription. However, many patients eventually develop resistance to tamoxifen and die of the cancer.

Activity of compound **12** was also tested against tamoxifen-resistant MCF-7 to find whether inhibition of HAT can affect growth of these cells. As summarized in Table 2, compound **12** exhibited strong antiproliferative activity against parent MCF-7 cells (EC₅₀ = 2.8 µM) as well as tamoxifen-resistant cells (EC₅₀ = 3.4 µM). These results show that inhibition of p300-mediated histone acetylation can inhibit growth of MCF-7 breast cancer cells and such activity is independent upon the status of tamoxifen resistance, suggesting the p300 HAT inhibitor could have potential clinical applications in breast cancer therapy.

**Table 2. Antiproliferative activities of compound 12.**

| | EC₅₀ (µM) |
|---|---|
| MCF-7 | 2.8 ± 0.1 |
| MCF-7 (Tam-R) | 3.4 ± 0.1 |
| PANC-1 | 1.0 ± 0.2 |
| MDA-PANC-28 | 2.8 ± 0.4 |
| Kasumi-1 | 2.6 ± 0.6 |

Moreover, p300 HAT activity has been reported to play important roles in pancreatic cancer and acute myeloid leukemia caused by oncogene RUNX1-ETO. Activity of compound **12** was evaluated against two pancreatic cancer cells PANC-1 and MDA-PANC-28 and RUNX1-ETO leukemia cell line Kasumi-1. As shown in Table 2, compound **12** showed potent activities against proliferation of the two pancreatic cancer cells with EC₅₀ values of 1.0 and 2.8 µM. It also inhibited growth of Kasumi-1 cells with an EC₅₀ of 2.6 µM.

### Example 1.9. Gene expression profiling

RNA sequencing was used to investigate how treatment with p300 HAT inhibitor **12** affects gene expression in ER⁺ breast cancer MCF-7 cells. Upon starvation (i.e., culturing in charcoal treated FBS without hydrophobic hormones including estrogens) for 7 days, MCF-7 cells were supplemented with estradiol (10 nM), followed by treatment with compound **12** for 2 days. Total RNA from three groups of MCF-7 cells, including starved, control (with estradiol) and treated (with estradiol and compound **12**) groups, was purified, prepared and sequenced. RNA sequencing data were mapped onto the human genome and gene expression profiles were determined and normalized. Genes with significantly changed expression levels were determined using a two-sided parametric t-test with the p value of <0.05. Gene set enrichment analysis (GSEA)(31) was used to analyze significant gene expression changes between these three groups of MCF-7 cells.

First, to determine whether the observed cellular activities of compound **12** are due to inhibition of p300 HAT, we used GSEA to compare the transcriptional profile of the **12**-treated MCF-7 cells with a publicly available p300 transcriptional signature (GSE31873), which was derived from siRNA-mediated p300 knockdown in C4-2B prostate cancer cells.

As shown in **FIG. 7A****,** there is a strong concordance between the two transcriptional signatures: Genes suppressed by compound **12** were strongly enriched among those suppressed by p300 knockdown, with normalized enrichment score (NES) of -3.32 and *p* value of <0.001. This result demonstrates that treatment with the p300 HAT inhibitor mimics the transcriptional footprint of p300 depletion and supports that p300 HAT is the cellular target of compound **12.**

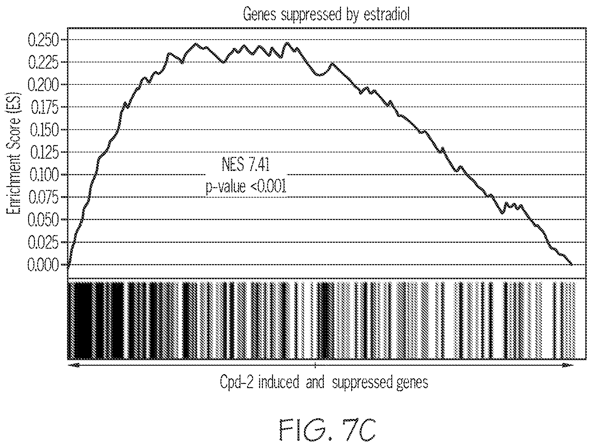

Next, activity of compound **12** in the ER signaling pathway was analyzed. As compared to starved cells, supplementation with estradiol potently caused upregulation as well as downregulation of several publicly available ER-related gene signatures in the control group of MCF-7 cells. Importantly, treatment with compound **12** counteracted such estrogen-induced gene transcription: The gene set upregulated by estradiol was strongly downregulated upon treatment with **12** (**FIG**. **7B**, NES = -9.19 and *p* < 0.001), and the genes suppressed by estradiol were significantly upregulated by compound **12** (**FIG. 7C****,** NES = 7.41 and *p* < 0.001). These results are consistent with previous studies showing p300 HAT is essential for ER-mediated gene expression, and demonstrate that pharmacological inhibition of p300 HAT can offset the effects of estrogen in gene regulation.

Moreover, Applicant investigated gene expression changes caused by compound **12** in the context of cancer biology and therapy. As shown in **FIG. 7D****,** global gene expression profiling analysis revealed that inhibition of p300 HAT by compound **12** counteracted estradiol and caused significant suppression of a number of estrogen-induced, cancer-related gene sets, including E2F and c-Myc gene signatures as well as those involved in cell proliferation, cell cycle, mitosis, DNA replication, DNA repair and self-renewal (stemness). Specifically for breast cancer, treatment with compound **12** downregulated a number of gene signatures that have previously been shown to be upregulated in multiple breast cancer patient datasets (as compared to normal breast tissues), associated with poor prognosis, cancer progression, invasion and relapse (**FIG. 7E**). Collectively, these GSEA results show the importance of p300 HAT in breast and other types of cancer, as well as the perspective for pharmacological inhibition of p300 HAT (by e.g., compound **12**) in breast cancer therapy.

### Example 1.10. Discussion

Histone acetylation by the HAT domain of p300/CBP has been found to play critical roles in many nuclear receptor-regulated signaling pathways, such as ER, AR and peroxisome proliferator-activated receptors (PPAR). Dysfunction of these gene transcription programs has been found in a number of diseases such as cancer and obesity. Moreover, p300/CBP has been found to directly contribute to oncogenesis (e.g., in RUNX1-ETO leukemia) or be part of a fusion oncogene due to chromosome translocation. Therefore, drug-like inhibitors of p300/CBP HAT are needed, which could be useful chemical probes and potential therapeutics for these indications. In addition, p300 and CBP are large proteins (~2,400 amino acids) with multiple other domains playing important physiological roles. They also share a high degree of homology with many duplicate functions. Given these two points, cell-permeable, small-molecule inhibitors of the HAT domain of p300/CBP are particularly useful for studying biological functions of p300/CBP HAT, because observed activities by genetic knockdown/knockout of p300 or CBP might not be relevant.

Compound screening followed by medicinal chemistry studies have led to the discovery of tri-substituted thiophene compound **12** that is a novel, small molecule inhibitor with an IC₅₀ of 620 nM against p300 HAT and 1.2 µM against CBP HAT. Compound 2 did not significantly inhibit two other major human HATs PCAF and Myst3 at 20 µM, showing a high selectivity. Biochemical studies showed that compound **12** is competitive against the substrate histone and non-competitive against the cofactor Ac-CoA, showing a distinct mode of inhibition from previous studied inhibitors C646 and A-485. Docking studies of **12** into the histone binding pocket of the p300 HAT structure provided possible binding models of the inhibitor in the protein.

In addition, compound **12** was found to be cell-permeable and inhibit cellular acetylation at several histone lysine residues, with H3K27 being the most sensitive substrate. With EC₅₀ of ~3 µM, compound **12** strongly inhibited proliferation of ER+ breast cancer cell line MCF-7, regardless of its sensitivity or resistance to the commonly used estrogen antagonist tamoxifen, suggesting the potential therapeutic application of this compound in the context of endocrine resistance. Compound **12** also exhibited strong activity against growth of several pancreatic cancer cells and RUNX1-ETO leukemia cell Kasumi-1, in which p300 HAT is known to be important.

Global gene transcription profiling was performed to investigate the cellular target of compound **12** as well as the mechanism for its observed biological activities in MCF-7 cells. First, the overall transcriptional changes upon treatment with compound **12** recapitulated those caused by siRNA-mediated p300 knockdown. This result, together with its inhibitory activity in cellular histone lysine acetylation, support that p300 HAT is the cellular target of compound **12.** Second, inhibition of p300 HAT activity by compound **12** significantly offset estrogen-induced gene expression, showing p300 HAT is essential for the ER activity. Moreover, treatment with compound **12** strongly downregulated a number of (general) cancer-related gene signatures, as well as gene sets that have been identified in the clinic for the progression, invasion, relapse and poor prognosis of breast cancer. These results, together with the compound's potent antiproliferative activities against several types of cancer cells, suggest that pharmacological inhibition of p300 HAT is a useful therapeutic approach to cancer treatment. In conclusion, Applicant's results demonstrate that compound **12** is not only a useful small-molecule probe for biological studies of p300/CBP HAT, but also a novel pharmacological lead for further drug development targeting breast and other types of cancer.

### Example 1.11. Compound synthesis and characterization

Compound synthesis and characterization can be found in the Supplementary Information for the following reference: J. Med. Chem. 2020, 63, 9, 4716-4731. Compound synthesis and characterization can be found in priority U.S. Provisional Patent Application No. 63/007,711.

### Example 1.12. Inhibition of p300-HAT and other HATs

The HAT domain (1195-1673) of human p300 was cloned, inserted into pGEX-KG vector and the DNA sequence was verified by sequencing. The p300-HAT expression plasmid was transformed into *E. coli* BL21-CodonPlus strain (Agilent) and cultured at 37 °C in LB medium containing ampicillin (50 µg/mL) and chloramphenicol (34 µg/mL). After the optical density of the bacterial culture reached ~0.9 at 600 nm, p300 HAT expression was induced by adding 300 µM isopropylthiogalactoside (IPTG) at 18 °C for 48 hours. Cells were collected, lysed, centrifuged for 20 min at 20,000 rpm. The supernatant was subjected to column chromatography with glutathione sepharose resins. The recombinant GST-p300-HAT fusion protein was obtained in ~90% purity (SDS-PAGE) by elution with 10 mM of glutathione solution, which was further purified using a Superdex 200 gel filtration column chromatography. CBP, PCAF and Myst3 HATs were obtained using similar methods.

To determine inhibitory activity, a compound with concentrations ranging from 100 nM to 10 µM was incubated with p300-HAT (10 nM) in 20 µL of 50 mM phosphate buffer (pH = 7.0) containing 0.01% Brij-35 for 10 min at 25 °C. Histone H3 peptide (1-21) (20 µM) and Acetyl-CoA (1 µM ³H-Ac-CoA and 19 µM Ac-CoA) were added to initiate the reaction. After 30 min at 25 °C, the reaction was stopped by adding 6 N formic acid (5 µL). 20 µL of reaction mixture was then transferred to a small piece of P81 filter paper (Whatman) that binds histone H3 peptide. The filter paper was washed 3x with 50 mM NaHCO₃, dried, and transferred into a scintillation vial containing 2 mL of scintillation cocktail. Radioactivity on the filter paper was measured with a Beckman LS-6500 scintillation counter. IC₅₀ values were obtained by using a standard sigmoidal dose response curve fitting in Prism (version 5.0, GraphPad Software, Inc., La Jolla, CA). IC₅₀ values were the mean values from at least three experiments.

### Example 1.13. Alpha assay

Applicant utilized an assay to investigate whether compound **12** can disrupt the binding of p300 HAT and histone H4 peptide. In brief, the assay was performed in a 384-well plate using His6-tagged P300 HAT (125 nM) coated nickel chelate acceptor beads (5 µL, Perkin Elmer), biotinylated H4 peptide [SGRGKGGKGLGKGGAKRHRKVLRGG-K(Biotin)-NH2] (30 nM) coated streptavidin donor beads (5 µL, Perkin Elmer), and varying concentrations of **12** in a PBS buffer with 0.5 % BSA (final volume of 25 µL). Upon incubation for 1h, Alpha signal was determined (laser excitations at 680 nm and reading at 570 nm) using a Tecan Spark microplate reader. The IC₅₀ values were determined using the sigmoidal dose-response fitting in the program of Prism 5.0 (GraphPad).

### Example 1.14. Molecular modeling

Docking studies were performed with Schrödinger small-molecule drug discovery software suite (Schrödinger, LLC, New York, NY, 2017). The crystal structure of p300-HAT in complex with Ac-CoA (PDB: 4PZS) was prepared using the module "protein preparation wizard" in Maestro with the default protein parameters. Hydrogen atoms were added and water molecules were extracted. Ac-CoA was included in the protein structure for docking. Hydrogen bonds were optimized, the partial charges were assigned, and the protein structure was energy-minimized using OPLS-2005 force field. A receptor grid was generated using the program Glide without any constraints. Compound **11** was constructed, energy-minimized using OPLS-2005 force field in Maestro and then docked into the prepared protein structure using Glide (docking parameters: standard-precision and dock flexibly).

### Example 1.15. Western blot

About 10⁶ Kasumi-1 cells per well were incubated with compound **11** at 0, 5 and 10 µM for 12 hours. Histone proteins were extracted using EpiQuik total histone extraction kit (Epigentek), according to the manufacturer's protocol. Equal amounts of histones (2 µg) were separated on SDS-PAGE and transferred to PVDF membranes. The blots were probed with primary antibodies against H3K9Ac, H3K18Ac, H3K27Ac and H3 (Cell Signaling), followed by anti-rabbit IgG (Thermo Scientific) secondary antibodies.

### Example 1.16. Cell growth inhibition

The anti-proliferation assays for non-breast cancer cells were done using a previously developed assay. In brief, for Kasumi-1 cells, 10⁶ cells per well were added into 96-well plates and cultured with increasing concentrations of a compound in RPMI-1640 medium supplemented with 20% fetal bovine serum and penicillin (100 U/mL) and streptomycin (100 µg/mL) at 37 °C in a 5% CO₂ atmosphere with 100% humidity. For solid tumor cells, 10⁵ cells per well were added into 96-well plates and cultured in Dulbecco's Modified Eagle's Medium (DMEM) supplemented with 10% fetal bovine serum and penicillin (100 U/mL) and streptomycin (100 µg/mL) overnight. Upon addition of increasing concentrations of a compound, cells were incubated for 5 days. Cell viability was assessed by using an XTT assay kit (Roche) for leukemia cells or an MTT assay (Sigma) for attachment cells. For the breast cancer MCF-7 cells, culture and inhibition assays were performed according to our previous methods in Ref. 25 and 26. Compound EC₅₀ values were calculated from dose response curves using Prism 5.0.

### Example 1.17. RNA extraction

Upon starvation (culturing in phenol red-free DMEM medium with 10% charcoal treated FBS to remove hydrophobic hormones such as estrogens) for 7 days, MCF-7 cells were treated with the p300 inhibitor (6 µM) or DMSO control, in the presence or absence of (10 nM) estradiol (E2) for 48 hours. Total RNA was extracted using RNeasy Plus Mini Kit (Qiagen), according to the manufacturer's instruction.

### Example 1.18. Library Preparation

The RNA integrity for each sample was assessed with a RNA 6000 Nano chip on a 2100 Bioanalyzer (Agilent; Santa Clara, CA). The average RNA integrity score for the sample set was 6.48. Sequencing libraries were prepared using the TruSeq Stranded Total RNA Library Prep Kit (Illumina, Inc; San Diego, CA). Concisely, ribosomal RNA (rRNA) was depleted from total RNA where the remaining (ribo-depleted RNA) was purified, fragmented for 7.5 minutes, and primed for cDNA synthesis. Blunt-ended cDNA was generated after first and second strand synthesis. Adenylation of the 3' blunt-ends was followed by adapter ligation prior to the enrichment of the cDNA fragments. Final library quality control was carried out by measuring the fragment size on a DNA1000 chip on a 2100 BioAnalyzer (Agilent; Santa Clara, CA). The average library yielded an insert bp size of 258. The concentration of each library was determined by quantitative PCR (qPCR) by the KAPA Library Quantification Kit for Next Generation Sequencing (KAPA Biosystems; Woburn, MA).

### Example 1.19. Sequencing

Libraries were normalized to 50 nmol/L in 10 mM Tris-Cl, pH8.5 with 0.1% Tween 20 then pooled evenly. The 50 nmol/L library pool was diluted to 2 nmol/L with 10 mM Tris-Cl, pH8.5 with 0.1% Tween 20. The pooled libraries were denatured with 0.05N NaOH and diluted to 20 pmol/L. Cluster generation of the denatured libraries was performed according to the manufacture's specifications (Illumina, Inc; San Diego, CA) utilizing the HiSeq Rapid PE Cluster Kit v2 chemistry and flow cell. Libraries were clustered appropriately with a 1% PhiX spike-in. Sequencing-by-synthesis (SBS) was performed on a HiSeq 2500 utilizing v2 chemistry with paired-end 101 bp reads and a 6 bp index read culminating in an average output of 20 million paired-end reads (or 40 million total reads) per sample. Sequence read data were processed and converted to FASTQ format by Illumina BaseSpace analysis software (v2.0.13).

### Example 1.20. Bioinformatics Analysis

RNAseq data files were trimmed using Trim Galore!. Then the data was mapped using Hisat2 onto the human reference genome build UCSC hg38/GRCh38and sorted using samtools. Gene expression was assessed and fragments per kilobase of transcript per million (FPKM) values were determined using Stringtie. Gene expression profiles were normalized using the quantile normalization method. Significantly changed genes were determined using a two-sided parametric t-test, with significance assessed at p<0.05, only genes for which at least one sample exceeded 1 FPKM in expression were considered. Enriched pathways were inferred using the Gene Set Enrichment (GSEA) method, and the Molecular Signature Database (MSigDB) pathway collection.
Without further elaboration, it is believed that one skilled in the art can, using the description herein, utilize the present disclosure to its fullest extent. The embodiments described herein are to be construed as illustrative and not as constraining the remainder of the disclosure in any way whatsoever.

## Claims

1. A composition comprising a compound, wherein the compound is selected from the group consisting of: 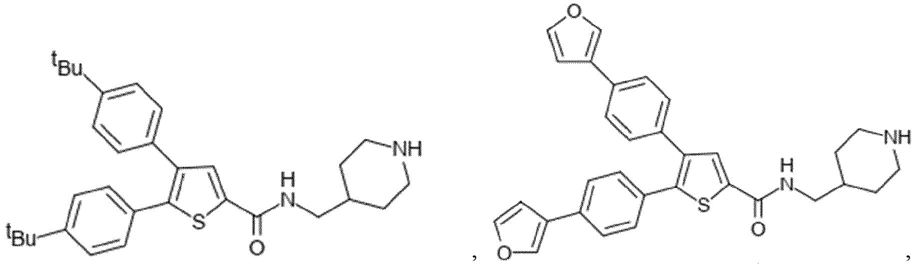 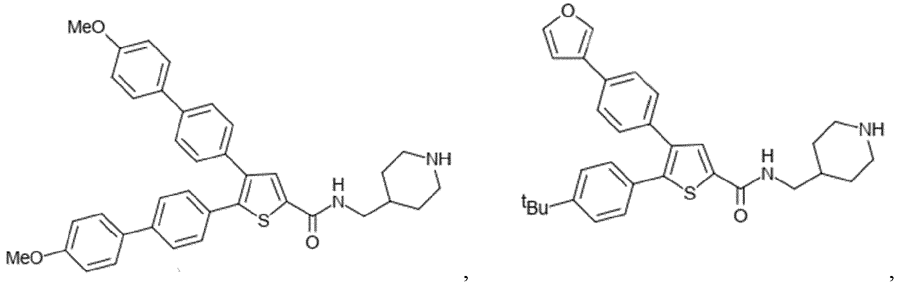 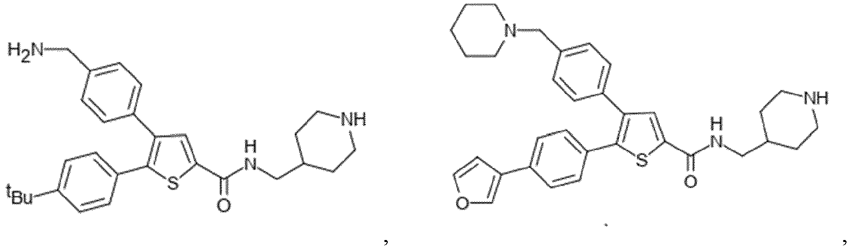 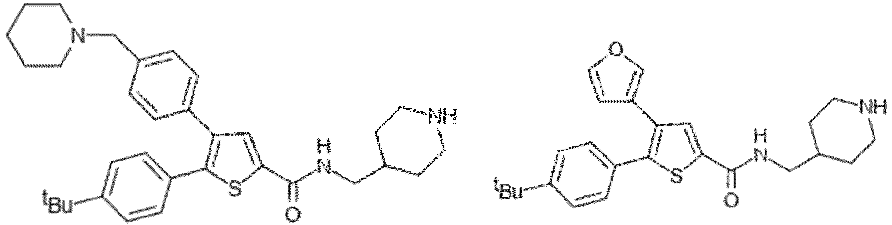 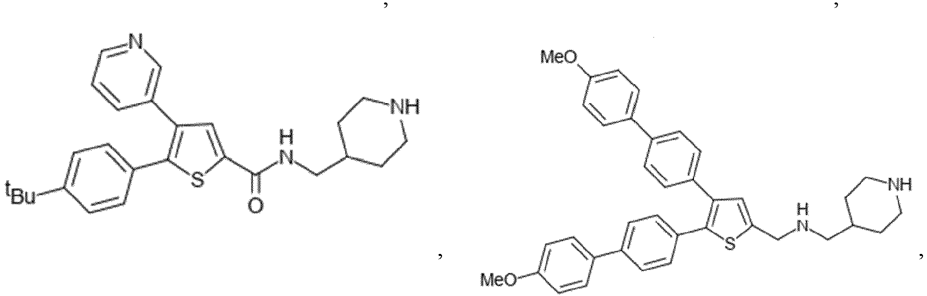 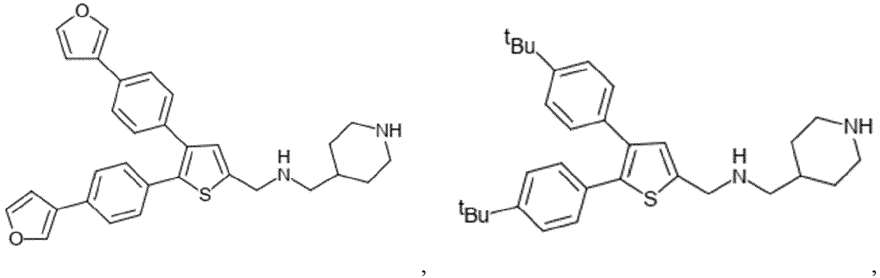 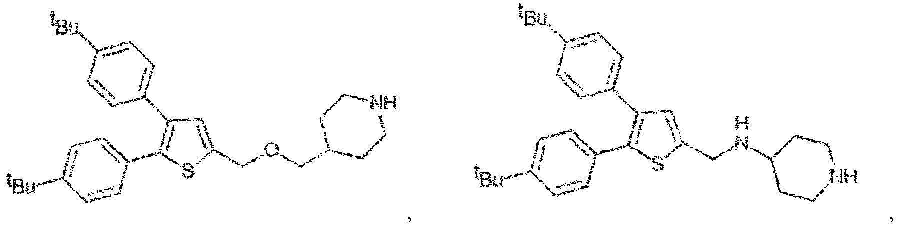 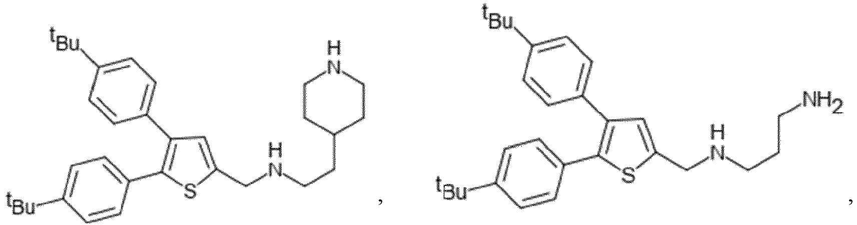 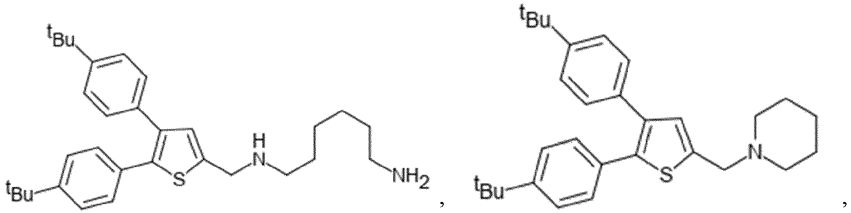 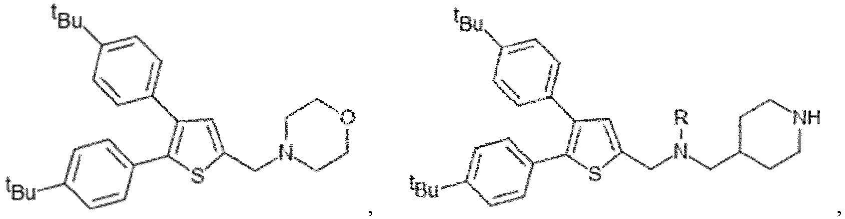 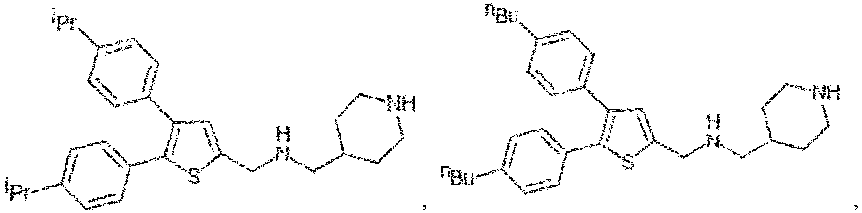 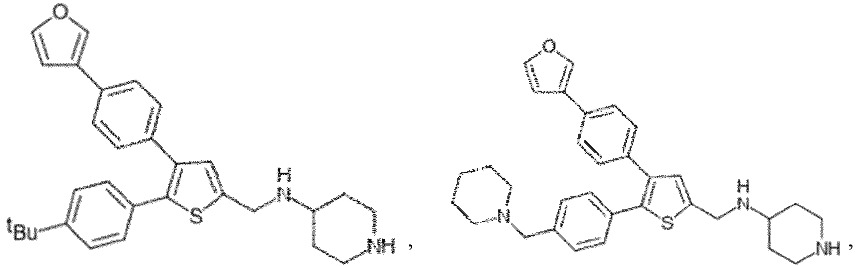 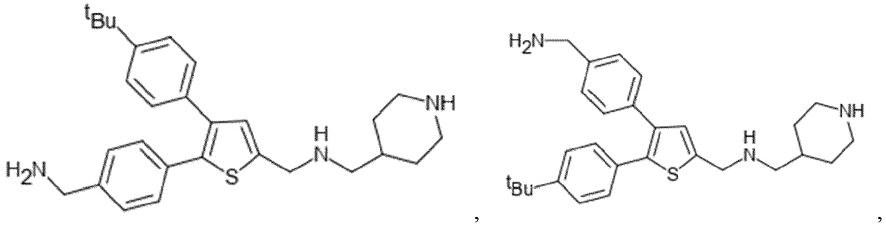 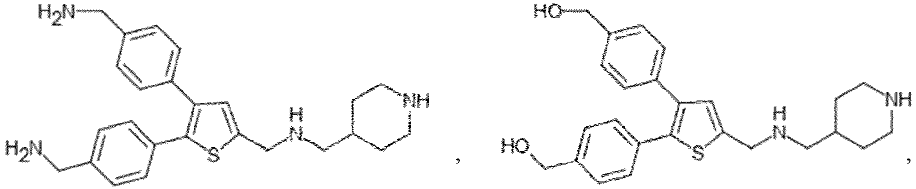 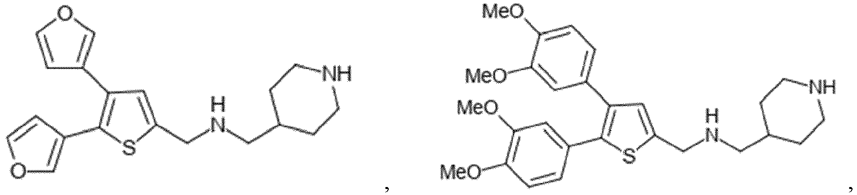 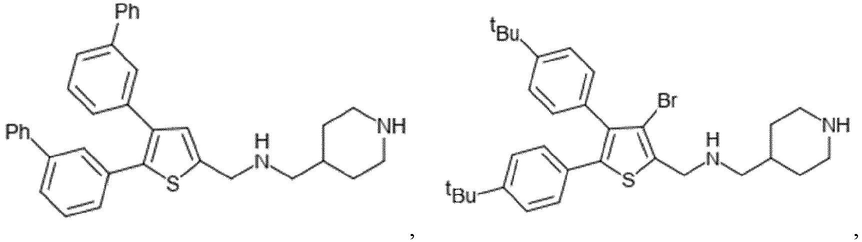 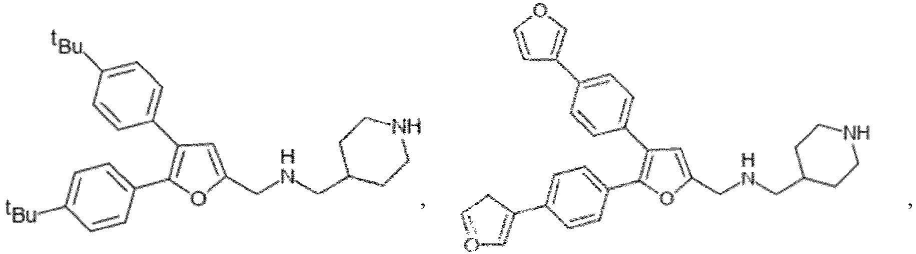 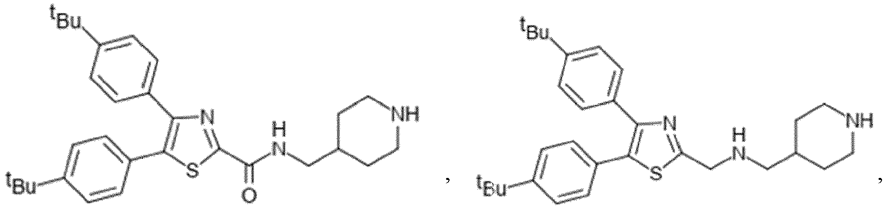 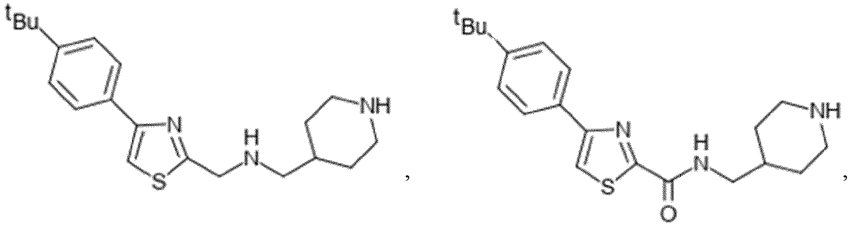 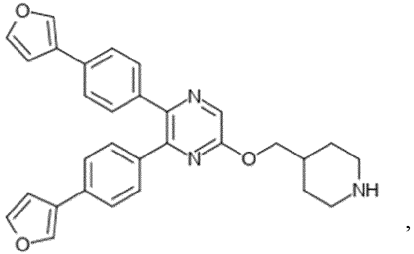 wherein R is Me or CHO.

2. A composition comprising a compound of claim 1 for use in treatment of cancer in a subject.

3. The composition of claim 1 or the composition for use of claim 2, wherein the compound comprises the following structure:

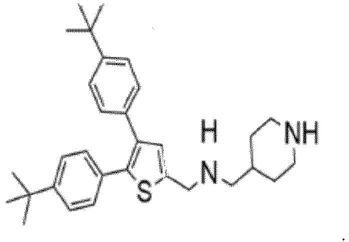

4. The composition of claim 1 or the composition for use of claim 2, wherein the compound comprises the following structure:

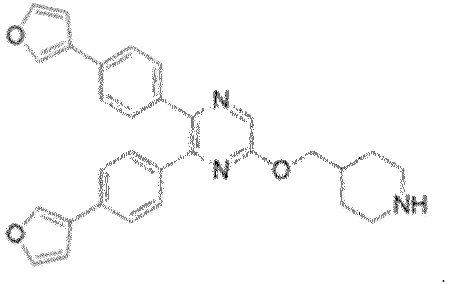

5. The composition for use of any one of claims 2 to 4, wherein the compound inhibits histone acetyl transferase activity of a protein.

6. The composition for use of any one of claims 2 to 5, wherein the cancer is selected from the group consisting of cancers associated with p300 overexpression, cancers associated with CBP overexpression, breast cancer, prostate cancer, pancreatic cancer, leukemia, acute myeloid leukemia, and combinations thereof.

7. A method of inhibiting the histone acetyl transferase activity of a protein, said method comprising:
exposing *in vitro* the protein to a composition as claimed in any one of claims 1, 3 and 4.

8. The composition for use of claim 5 or the method of claim 7, wherein the protein is selected from the group consisting of E1A binding protein p300 (p300), CREB (cAMP-response element binding protein) binding protein (CBP), and combinations thereof.

9. The composition for use of claim 5 or the method of claim 7, wherein the compound inhibits the histone acetyl transferase activity of the protein with an IC50 value ranging from 500 nm to 15 mM.

## Patentansprüche

1. Eine Zusammensetzung, die eine Verbindung beinhaltet, wobei die Verbindung ausgewählt ist aus der Gruppe, die aus Folgendem besteht: 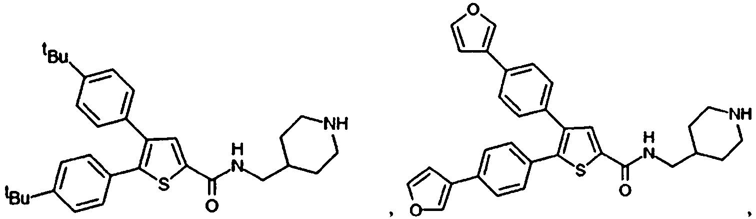 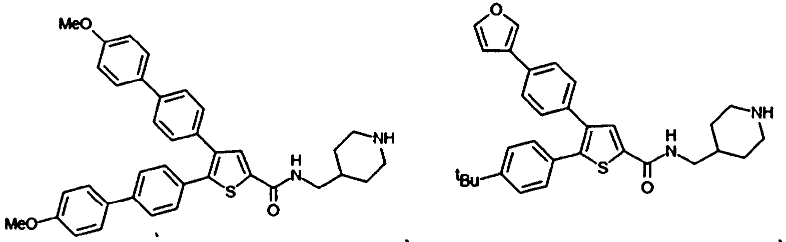 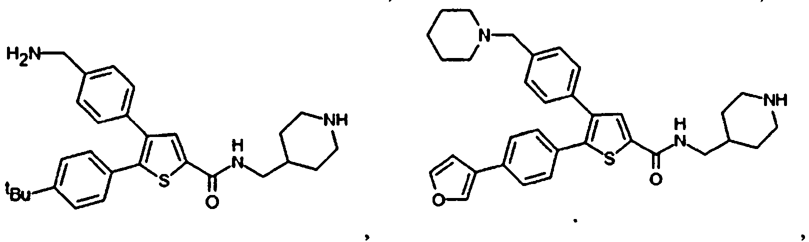 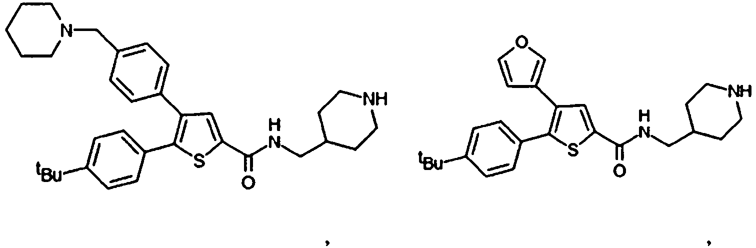 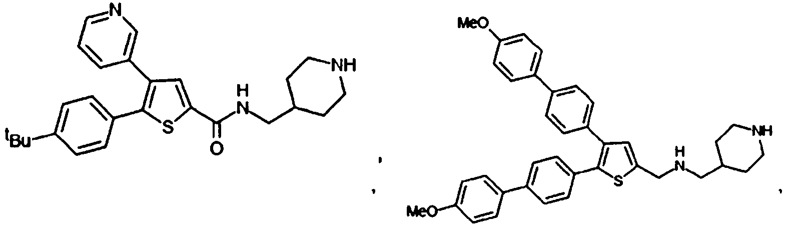 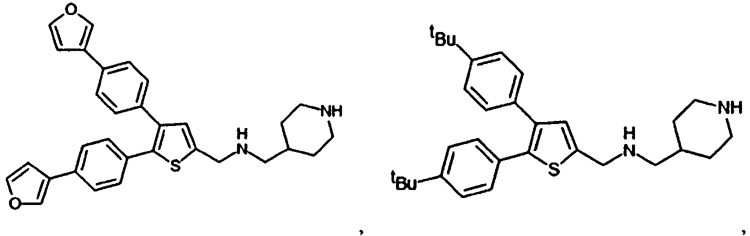 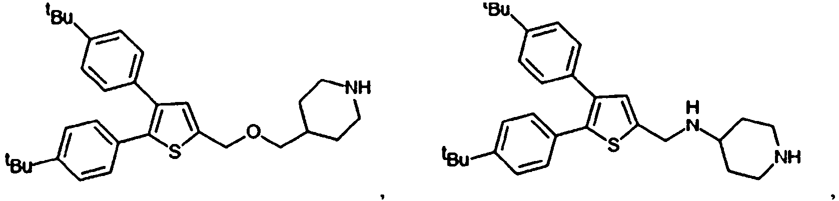 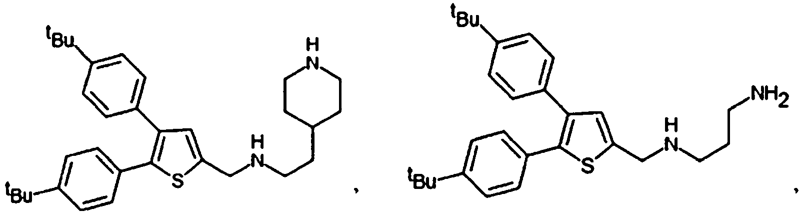 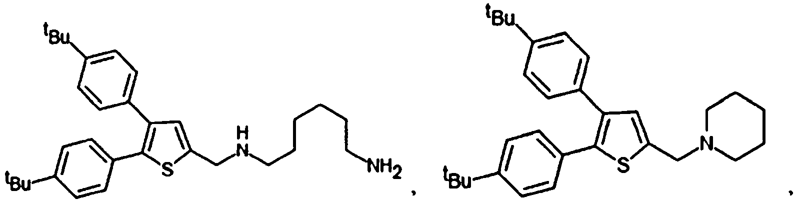 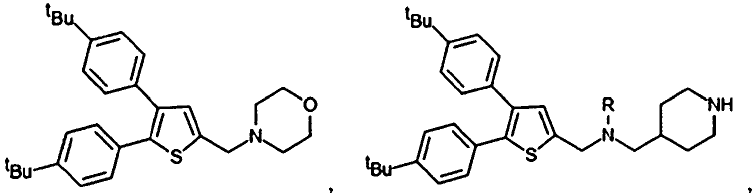 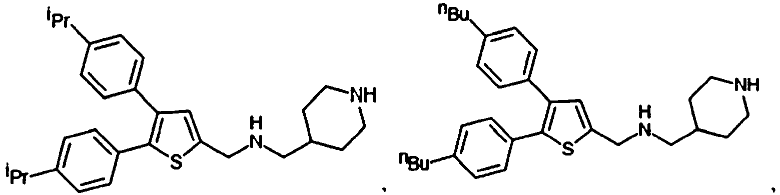 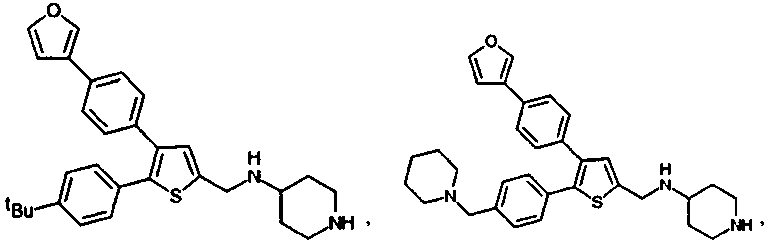 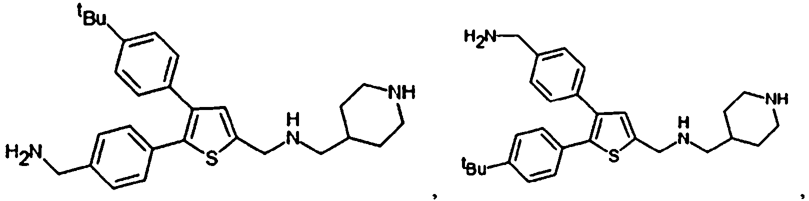 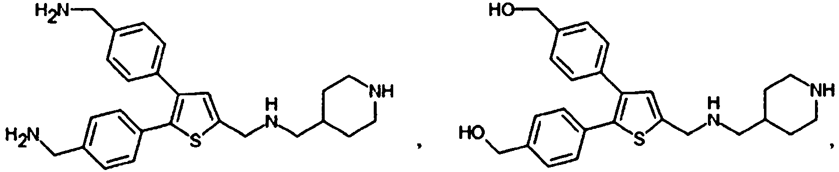 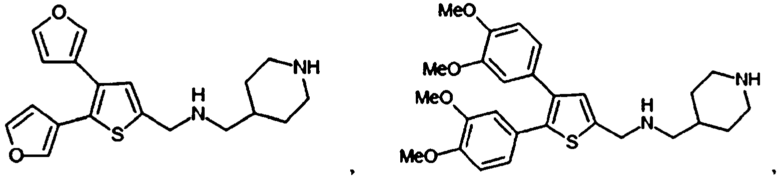 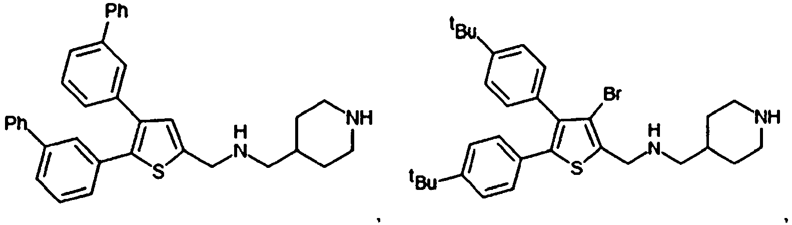 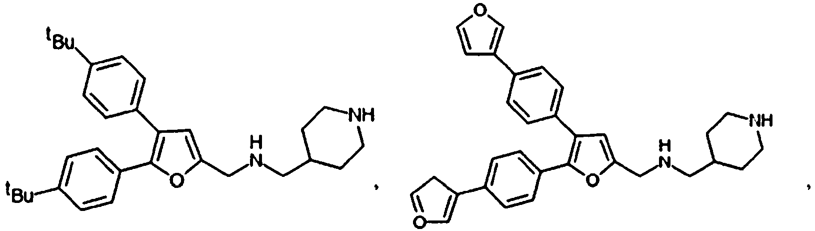 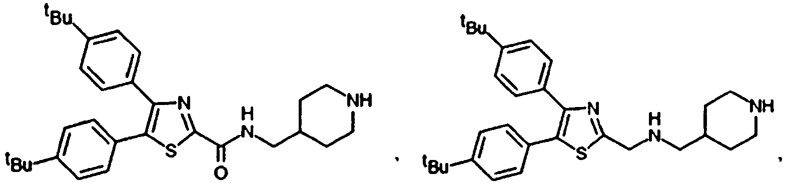 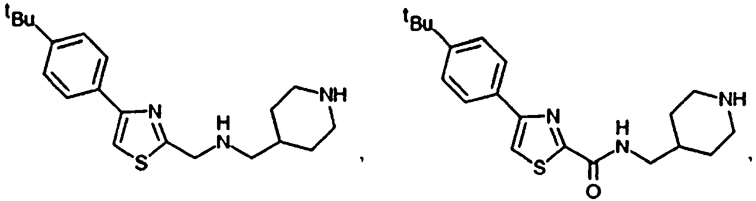 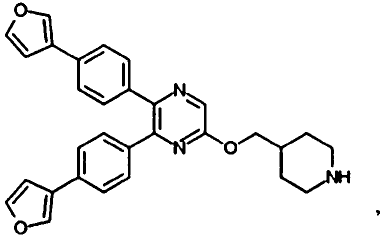 wobei R Me oder CHO ist.

2. Eine Zusammensetzung, die eine Verbindung gemäß Anspruch 1 zur Verwendung bei der Behandlung von Krebs in einem Individuum beinhaltet.

3. Zusammensetzung gemäß Anspruch 1 oder Zusammensetzung zur Verwendung gemäß Anspruch 2, wobei die Verbindung die folgende Struktur beinhaltet:

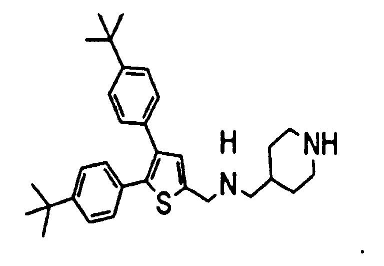

4. Zusammensetzung gemäß Anspruch 1 oder Zusammensetzung zur Verwendung gemäß Anspruch 2, wobei die Verbindung die folgende Struktur beinhaltet:

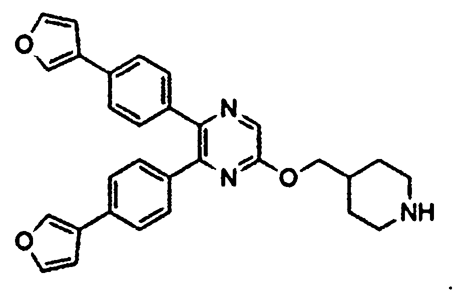

5. Zusammensetzung zur Verwendung gemäß einem der Ansprüche 2 bis 4, wobei die Verbindung die Histon-Acetyltransferase-Aktivität eines Proteins hemmt.

6. Zusammensetzung zur Verwendung gemäß einem der Ansprüche 2 bis 5, wobei der Krebs ausgewählt ist aus der Gruppe, die aus Krebsen, die mit p300-Überexpression assoziiert sind, Krebsen, die mit CBP-Überexpression assoziiert sind, Brustkrebs, Prostatakrebs, Bauchspeicheldrüsenkrebs, Leukämie, akuter myeloischer Leukämie und Kombinationen davon besteht.

7. Ein Verfahren zum Hemmen der Histon-Acetyltransferase-Aktivität eines Proteins, wobei das Verfahren Folgendes beinhaltet:
*In-vitro*-Aussetzen des Proteins gegenüber einer Zusammensetzung gemäß einem der Ansprüche 1, 3 und 4.

8. Zusammensetzung zur Verwendung gemäß Anspruch 5 oder Verfahren gemäß Anspruch 7, wobei das Protein ausgewählt ist aus der Gruppe, die aus EIA-Bindungsprotein p300 (p300), CREB(cAMP-response element binding protein)-Bindungsprotein (CBP) und Kombinationen davon besteht.

9. Zusammensetzung zur Verwendung gemäß Anspruch 5 oder Verfahren gemäß Anspruch 7, wobei die Verbindung die Histon-Acetyltransferase-Aktivität des Proteins mit einem IC50-Wert im Bereich von 500 nm bis 15 mM hemmt.

## Revendications

1. Une composition comprenant un composé, dans laquelle le composé est choisi dans le groupe constitué de : 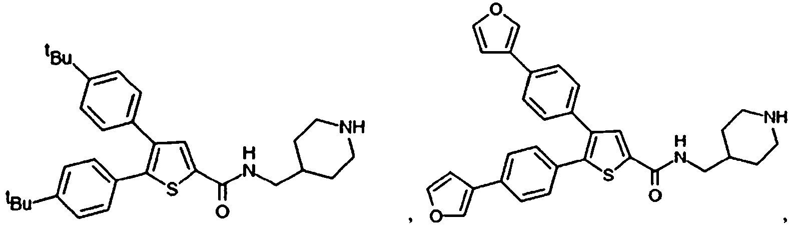 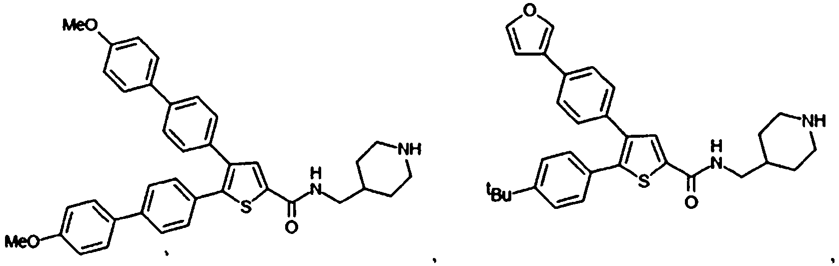 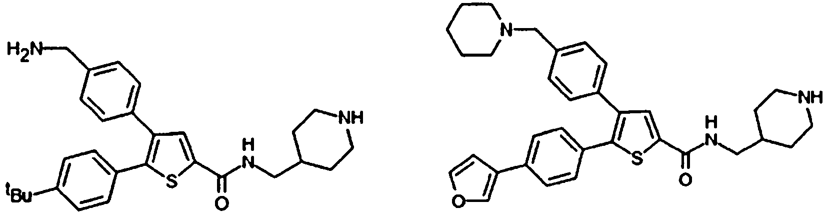 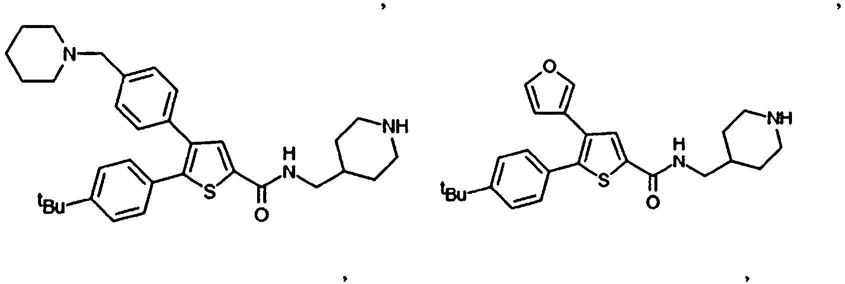 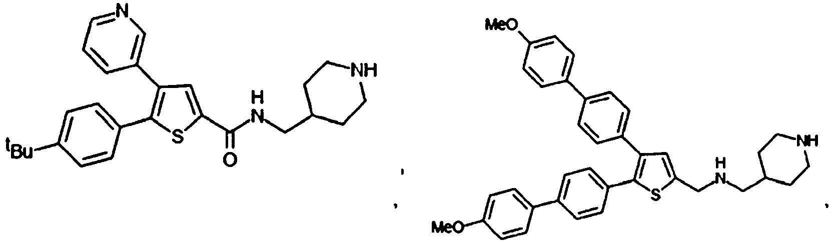 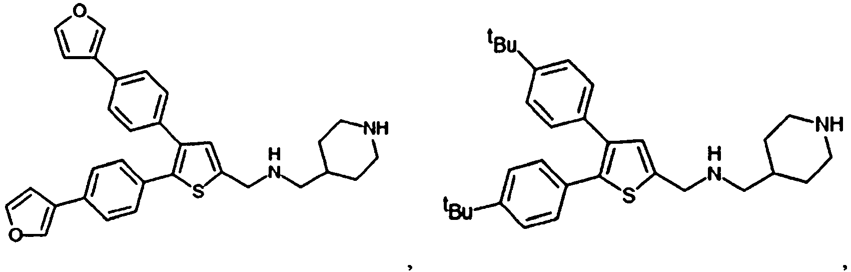 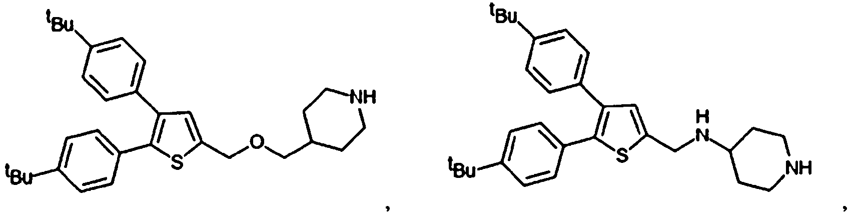 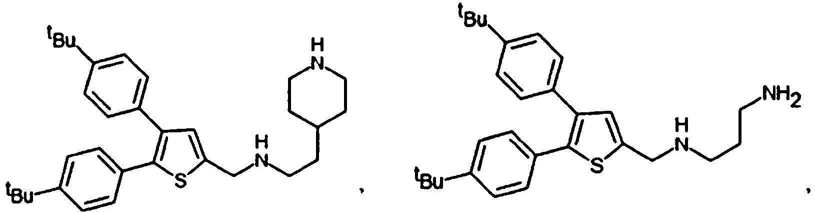 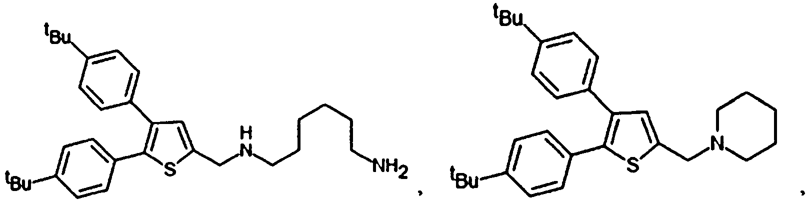 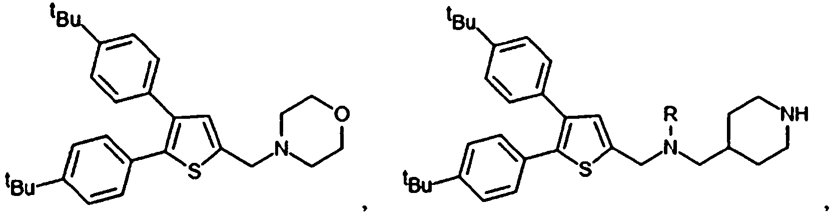 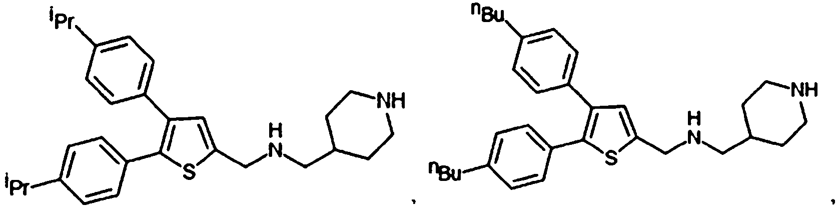 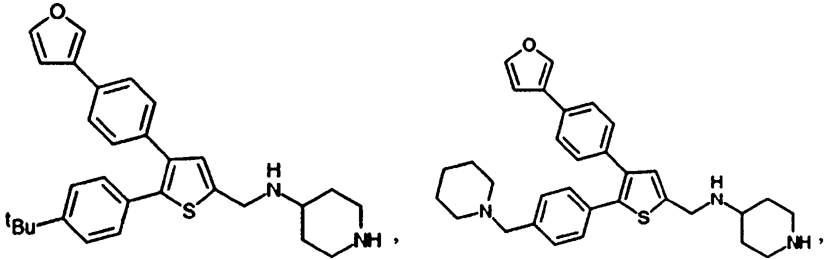 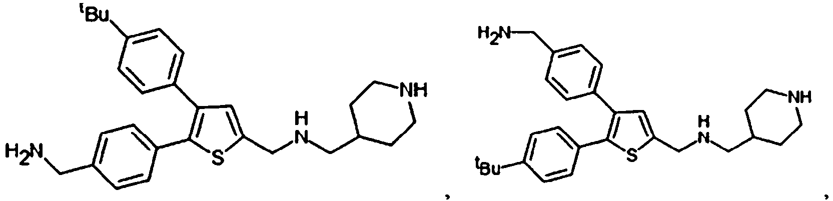 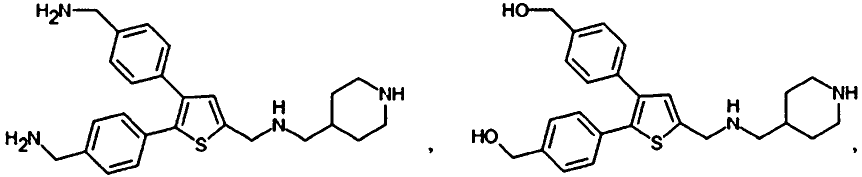 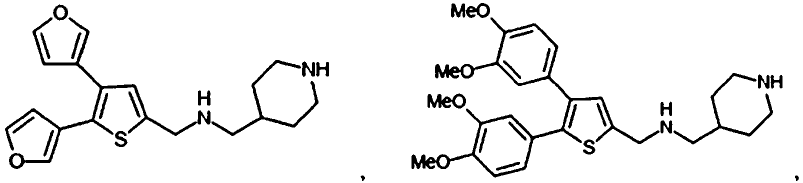 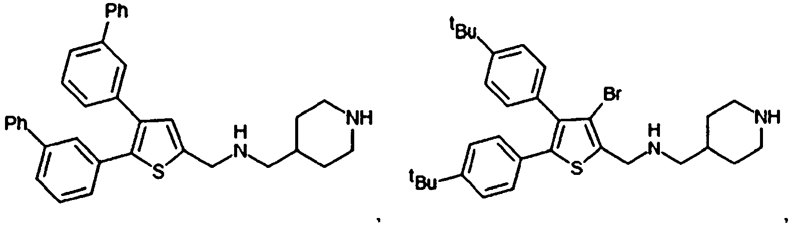 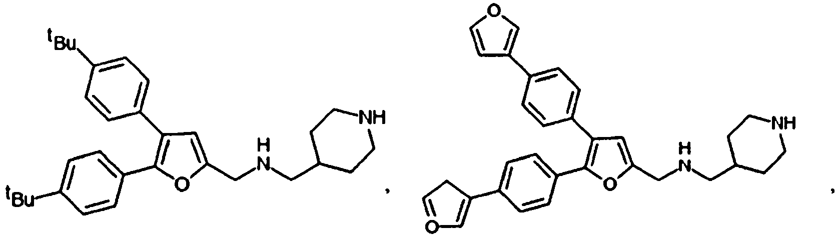 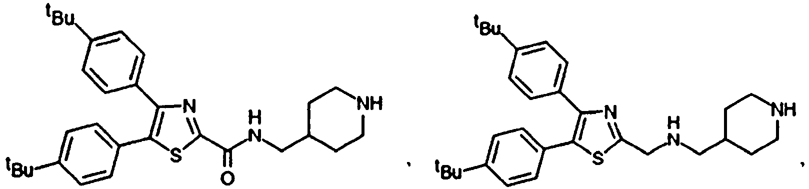 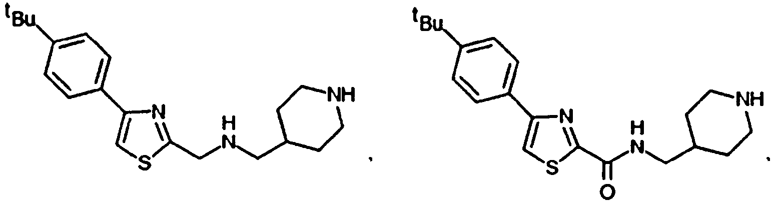 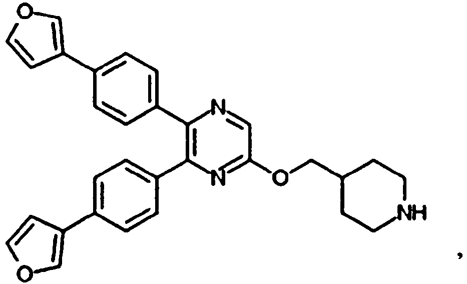 où R est Me ou CHO.

2. Une composition comprenant un composé de la revendication 1 pour son utilisation dans le traitement d'un cancer chez un sujet.

3. La composition de la revendication 1 ou la composition pour son utilisation selon la revendication 2, dans laquelle le composé comprend la structure suivante :

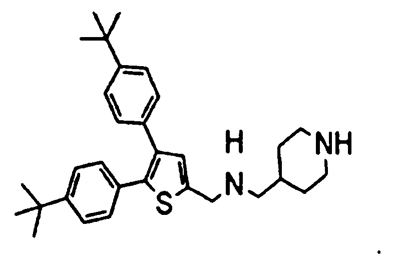

4. La composition de la revendication 1 ou la composition pour son utilisation selon la revendication 2, dans laquelle le composé comprend la structure suivante :

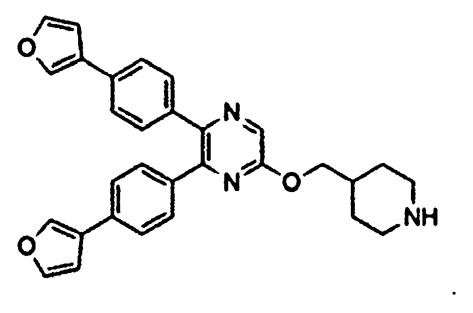

5. La composition pour son utilisation selon l'une quelconque des revendications 2 à 4, dans laquelle le composé inhibe l'activité histone acétyltransférase d'une protéine.

6. La composition pour son utilisation selon l'une quelconque des revendications 2 à 5, dans laquelle le cancer est choisi dans le groupe constitué de cancers associés à une surexpression de p300, de cancers associés à une surexpression de CBP, du cancer du sein, du cancer de la prostate, du cancer du pancréas, de la leucémie, de la leucémie myéloïde aiguë, et de combinaisons de ceux-ci.

7. Une méthode d'inhibition de l'activité histone acétyltransférase d'une protéine, ladite méthode comprenant :
l'exposition *in vitro* de la protéine à une composition telle que revendiquée dans l'une quelconque des revendications 1, 3 et 4.

8. La composition pour son utilisation selon la revendication 5 ou la méthode de la revendication 7, dans laquelle la protéine est choisie dans le groupe constitué de la protéine de liaison à E1A p300 (p300), de la protéine de liaison à CREB (protéine de liaison à l'élément de réponse à l'AMPc) (CBP), et de combinaisons de celles-ci.

9. La composition pour son utilisation selon la revendication 5 ou la méthode de la revendication 7, dans laquelle le composé inhibe l'activité histone acétyltransférase de la protéine avec une valeur CI50 allant de 500 nm à 15 mM.
